# EUROPÄISCHE PATENTSCHRIFT

(11) **EP 2 173 742 B1**
(45) Veröffentlichungstag und Bekanntmachung des Hinweises auf die Patenterteilung: **23.11.2011**
(21) Anmeldenummer: 08774790.3
(22) Anmeldetag: 04.07.2008
(51) Int. Cl.: C07D 413/10, A01N 43/80

(54) **KRISTALLINE FORM DES [3-(4,5-DIHYDRO-3-ISOXAZOLYL)-2-METHYL-4-(METHYLSULFONYL)PHENYL]-(5-HYDROXY-1-METHYL-1H-PYRAZOL-4-YL)METHANONS**
CRYSTALLINE FORM OF [3-(4,5-DIHYDRO-3-ISOXAZOLYL)-2-METHYL-4-(METHYLSULFONYL)PHENYL]-(5-HYDROXY-1-METHYL-1H-PYRAZOL-4-YL)METHANONE
FORME CRISTALLINE DE LA [3-(4,5-DIHYDRO-3-ISOXAZOLYL)-2-MÉTHYL-4-(MÉTHYLSULFONYL)PHÉNYL]-(5-HYDROXY-1-MÉTHYL-1H-PYRAZOL-4-YL)MÉTHANONE

(30) Priorität: 06.07.2007 EP 07111981
(43) Veröffentlichungstag der Anmeldung: 14.04.2010
(73) Patentinhaber: BASF SE, 67056 Ludwigshafen (DE)
(72) Erfinder: GEBHARDT, Joachim, 67157 Wachenheim (DE); ERK, Peter, 67227 Frankenthal (DE); SAXELL, Heidi Emilia, 67316 Carlsberg (DE); KRÖHL, Thomas, 69198 Schriesheim (DE); BRATZ, Matthias, 67133 Maxdorf (DE)
(86) Internationale Anmeldenummer: PCT/EP2008/058712
(87) Internationale Veröffentlichungsnummer: WO 2009/007329

(56) Entgegenhaltungen:
- A. SCHÖNHAMMER, J. FREITAG, H. KOCH: "Topramezone - ein neuer Herbizidwirkstoff zur hochselektiven Hirse- und Unkrautbekämpfung in Mais" JOURNAL OF PLANT DISEASES AND PROTECTION; ZEITSCHRIFT FÜR PLFANZENKRANKHEITEN UND PFLANZENSCHUTZ, SONDERHEFT, 2006, Seiten 1023-1031, XP002500868 Stuttgart

## Beschreibung

Die vorliegende Erfindung betrifft kristalline Formen des [3-(4,5-Dihydro-3-isoxazolyl)-2-methyl-4-(methylsulfonyl)phenyl]-(5-hydroxy-1-methyl-1*H*pyrazol-4-yl)methanons, das auch unter dem Common Name Topramezon bekannt ist. Die Erfindung betrifft auch ein Verfahren zur Herstellung dieser kristallinen Formen sowie Formulierungen für den Pflanzenschutz, welche eine dieser kristallinen Formen des Topramezons enthalten.

Bei Topramezon handelt es sich um den herbiziden Wirkstoff der Formel I bzw. und das Tautomer der Formel I'.

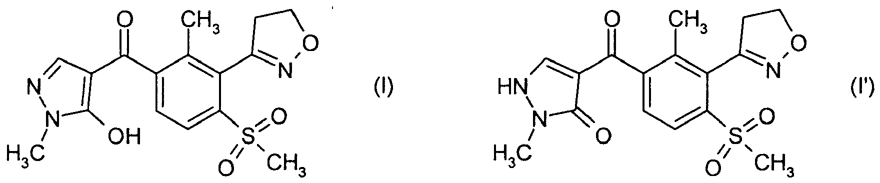

Topramezon und eine allgemeine Vorschrift zu seiner Herstellung sind aus der WO 98/31681 und WO 99/58509 bekannt. Kristallines Topramezone ist aus dem Journal of Plant Diseases and Protection, Zeitschrift für Pflanzenkrankheiten und Pflanzenschutz, Sonderheft, 1023 - 1031, 2006, ISSN1861-405, bekannt.

Für die Herstellung von Wirkstoffen in technischem Maßstab aber auch für die Formulierung von Wirkstoffen ist in vielen Fällen die Kenntnis über eine etwaige Existenz von kristallinen Modifikationen (auch als kristalline Formen bezeichnet) oder von Solvaten des jeweiligen Wirkstoffs, die Kenntnis der spezifischen Eigenschaften solcher Modifikationen und Solvate sowie von Methoden zu ihrer Herstellung von entscheidender Bedeutung. Eine Reihe von Wirkstoffen kann in verschiedenen kristallinen, aber auch amorphen Modifikationen vorliegen. In diesen Fällen spricht man von Polymorphie. Ein Polymorph ist eine feste, kristalline Phase einer Verbindung, die durch eine bestimmte, einheitliche Packung und Anordnung der Moleküle im Feststoff charakterisiert ist.

Verschiedene Modifikationen ein und desselben Wirkstoffs können teilweise verschiedene Eigenschaften aufweisen, beispielsweise Unterschiede in den folgenden Eigenschaften: Löslichkeit, Dampfdruck, Lösungsgeschwindigkeit, Stabilität gegenüber einer Phasenumwandlung in eine andere Modifikation, Stabilität beim Vermahlen, Suspensionsstabilität, optische und mechanische Eigenschaften, Hygroskopie, Kristallform und Phasenumwandlung in eine andere Modifikation, Stabilität beim Vermahlen, Suspensionsstabilität, optische und mechanische Eigenschaften, Hygroskopie, Kristallform und -größe, Filtrierbarkeit, Dichte, Schmelzpunkt, Zersetzungsstabilität, Farbe und teilweise auch chemische Reaktivität oder biologische Aktivität.

Eigene Versuche der Anmelderin, Topramezon durch Kristallisation in einen kristallinen Feststoff zu überführen, führten zunächst zu komplexen Gemischen verschiedener Kristallmodifikationen. Die Stabilität daraus hergestellter Formulierungen war nicht in allen Fällen zufriedenstellend.

Es wurde nunmehr überraschenderweise gefunden, dass durch bestimmte Verfahren eine bislang nicht bekannte kristalline, stabile Modifikation des Topramezons mit hoher Reinheit erhalten wird, welche die Nachteile der anderen festen Formen des Topramezons nicht aufweist. Diese Modifikation wird im Folgenden auch als Form I bezeichnet. Daneben wurden noch vier weitere kristalline Formen des Topramezons und drei kristalline Lösungsmittel-Solvate des Topramezons gefunden, die jedoch eine geringere Stabilität aufweisen. Diese Formen werden im Folgenden als Form II, Form III, Form IV, Form VIII, Form V-S, Form VI-S und Form VII-S bezeichnet.

Zudem ist die erfindungsgemäße kristalline Form I leichter zu handhaben als andere feste Formen des Topramezons, da sie bei der Herstellung in Form diskreter Kristalle oder Kristallite anfällt. Im Unterschied zu den nadelförmigen Kristallen der anderen Modifikationen des Topramezons fällt die Form I in kompakten, in der Regel blockförmigen Kristallen an, die sich leichter filtrieren lassen. Im Vergleich zu anderen festen Formen weist die Form I eine erhöhte Stabilität bezüglich einer Umwandlung in eine andere Form auf. Auch die Stabilität von Formulierungen, welche Topramezon in der Form I enthalten, ist deutlich höher als die Stabilität von Formulierungen, die andere feste Formen des Topramezons enthalten.

Der hier und im Folgenden verwendete Begriff "reine Form I" ist so zu verstehen, dass der Anteil der Modifikation Form I, bezogen auf die Gesamtmenge an Topramezon im Feststoff bzw. in der Formulierung, wenigstens 90 Gew.-% und insbesondere wenigstens 95 Gew.-% beträgt.

Dementsprechend betrifft ein erster Gegenstand der vorliegenden Erfindung die kristalline Form I des Topramezons. Gegenstand ist auch ein Topramezon, das zu wenigstens 90 Gew.-%, insbesondere zu wenigstens 95 Gew.-% aus der kristallinen Form I besteht.

Die erfindungsgemäße Form I kann mittels Röntgenpulverdiffraktometrie anhand ihres Beugungsdiagramms identifiziert werden. So zeigt ein bei 30 °C unter Verwendung von Cu-Kα-Strahlung (1,54178 Å) aufgenommenes Röntgenpulverdiffraktogramm wenigstens 5, häufig wenigstens 6, insbesondere wenigstens 7, und speziell alle der in der folgenden Tabelle 1 als 2θ-Werte, bzw. als Netzebenenabstände d, angegebenen Reflexe:

**Tabelle 1:**

| 2θ | d [Å] |
|---|---|
| 7,7 ± 0,2 | 11,46 ± 0,05 |
| 10,3 ± 0,2 | 8,58 ± 0,05 |
| 12,7 ± 0,2 | 6,96 ± 0,05 |
| 13,8 ± 0,2 | 6,42 ± 0,04 |
| 16,9 ± 0,2 | 5,24 ± 0,03 |
| 18,8 ± 0,2 | 4,72 ± 0,03 |
| 20,7 ± 0,2 | 4,28 ± 0,02 |
| 22,2 ± 0,2 | 4,00 ± 0,02 |
| 28,0 ± 0,2 | 3,19 ± 0,02 |
| 31,4 ± 0,2 | 2,81 ± 0,02 |

Untersuchungen an Einkristallen der Form I zeigen, dass die zugrunde liegende Kristallstruktur monoklin ist. Die Elementarzelle weist die Raumgruppe P2(1)/c auf. Die charakteristischen Daten der Kristallstruktur von Form I (bestimmt bei 20 °C) sind in der Tabelle 2 zusammengefasst.

**Tabelle 2: Kristallographische Eigenschaften der Form I**

| Parameter | Form I |
|---|---|
| Klasse | monoklin |
| Raumgruppe | P2(1)/c |
| a | 11,477(4) Å |
| b | 12,840(4) Å |
| c | 11,523(5) Å |
| α | 90 ° |
| β | 92,094(9)° |
| γ | 90 ° |
| Volumen | 1697,0(8) Å³ |
| Z | 4 |
| Dichte (berechnet) | 1,42 g/cm³ |
| R¹ ; ωR² | 0,09, 0,23 |
| Wellenlänge | 1,54178 Å |

| | |
|---|---|
| a,b,c = Kantenlänge der Elementarzelle α,β,γ = Winkel der Elementarzelle Z = Anzahl der Moleküle in der Elementarzelle | |

Form I zeigt ein Thermogramm mit charakteristischem Schmelzpeak. Der Schmelzpunkt, bestimmt als Beginn des Schmelzpeaks (onset), liegt typischerweise im Bereich von 218 bis 221 °C. Die Schmelzwärme beträgt etwa 126 J/g. Die hier angegebenen Werte beziehen sich auf mittels Differentialkalorimetrie (Differential Scanning Calorimetry: DSC, offener Tiegel aus Aluminium, Aufheizrate 10 K/min) ermittelte Werte.

Die Herstellung der erfindungsgemäßen Form I des Topramezons gelingt durch Kristallisation aus einer Lösung des Topramezons in einem geeigneten organischen Lösungsmittel. Geeignete Lösungsmittel zur Kristallisation der Form I sind C₁-C₄-Alkanole wie Methanol, Ethanol oder Isopropanol, weiterhin C₂-C₄-Alkandiole wie Glykol, weiterhin Aceton und deren Gemische mit Wasser.

Hierzu stellt man in einem ersten Schritt i) eine Lösung des Topramezons in einem der vorgenannten organischen Lösungsmittel bereit und bewirkt anschließend in einem zweiten Schritt ii) eine Kristallisation des Topramezons.

Die Konzentration an Topramezon in der zur Kristallisation eingesetzten Lösung hängt naturgemäß von der Art des Lösungsmittels und der Lösungstemperatur ab und liegt häufig im Bereich von 10 bis 400 g/L. Geeignete Bedingungen können vom Fachmann durch Routineexperimente ermittelt werden.

Vorzugsweise enthält die zur Kristallisation eingesetzte Lösung Topramezon in einer chemischen Reinheit von wenigstens 85 %, häufig wenigstens 90 %, insbesondere wenigstens 95 %, d. h. der Anteil organischer Verunreinigungen, die kein organisches Lösungsmittel sind, macht nicht mehr als 15 Gew.-%, häufig nicht mehr als 10 Gew.-% und insbesondere nicht mehr als 5 Gew.-%, bezogen auf das in dem Lösungsmittel gelöst vorliegende Topramezon, aus.

Die zur Kristallisation eingesetzte Lösung ist vorzugsweise im Wesentlichen frei von anderen als den genannten Lösungsmitteln. "Im Wesentlichen frei" bedeutet in diesem Zusammenhang, dass die Konzentration an anderen Lösungsmitteln in der Topramezon enthaltenden Lösung 10 Gew.-%, häufig 5 Gew.-%, bezogen auf die Gesamtmenge an Lösungsmittel, nicht überschreitet.

Die Lösung des Topramezons kann z. B. durch folgende Methoden bereitgestellt werden:
(1) Lösen des Topramezons, vorzugsweise einer von Form I verschiedenen Form, in einem der vorgenannten organischen Lösungsmittel, oder
(2) Herstellung des Topramezons durch eine chemische Umsetzung und Überführung des Reaktionsgemischs, gegebenenfalls nach Abtrennen von Reagenzien und/oder Nebenprodukten, in ein erfindungsgemäß geeignetes organisches Lösungsmittel.

Zur Herstellung der Lösung durch Lösen des Topramezons kann grundsätzlich jede bekannte Form des Topramezons eingesetzt werden. Häufig wird man amorphes Topramezon oder eine Mischung verschiedener kristalliner Modifikationen oder ein Gemisch aus amorphem und kristallinem Topramezon einsetzen. Geeignet sind auch kristalline Formen des Topramezons sowie deren Mischungen, z. B. die im Folgenden beschriebenen Formen II, III IV, V-S, VI-S oder VII-S sowie Mischungen dieser Formen.

Das Lösen des Topramezons erfolgt üblicherweise bei Temperaturen im Bereich von 20 bis 120 °C. In einer bevorzugten Ausführungsform der Erfindung erfolgt das Lösen des Topramezons bei erhöhter Temperatur, insbesondere bei wenigstens 50 °C, wobei die zum Lösen angewendete Temperatur naturgemäß den Siedepunkt des Lösungsmittels nicht überschreiten wird. Häufig erfolgt das Lösen bei Temperaturen im Bereich von 50 bis 120 °C.

Die Lösung des Topramezons kann auch dadurch bereitgestellt werden, dass man ein durch eine chemische Umsetzung erhaltenes Reaktionsgemisch, welches das Topramezon enthält, gegebenenfalls nach Abtrennen von Reagenzien und/oder Nebenprodukten, in ein erfindungsgemäß geeignetes organisches Lösungsmitte überführt. Dabei kann man so vorgehen, dass man die Umsetzung in einem organischen Lösungsmittel oder Lösungsmittelgemisch durchführt, das zumindest teilweise, vorzugsweise zu wenigstens 50 Gew.-% aus einem zur Kristallisation geeigneten Lösungsmittel besteht und, sofern erforderlich, eine Aufarbeitung durchführt, wobei man überschüssige Reagenzien und ggf. vorhandene Katalysatoren, ggf. vorhandenes, nicht geeignetes Lösungsmittel, z. B. Wasser und/oder Methanol entfernt. Die Herstellung einer Lösung des Topramezons durch chemische Umsetzung einer geeigneten Vorstufe des Topramezons, kann in Analogie zu den Methoden, die im eingangs zitierten Stand der Technik beschrieben sind, erfolgen, worauf hier in vollem Umfang Bezug genommen wird.

Die Kristallisation der Form I des Topramezons kann in folgender Weise bewirkt werden, z. B.
- durch Abkühlen der Lösung, welche das Topramezon gelöst enthält,
- durch Einengen der Lösung, welche das Topramezon gelöst enthält, oder
- durch eine Kombination der vorgenannten Maßnahmen.

Die Kristallisation wird in der Regel soweit geführt, dass wenigstens 80 Gew.-%, vorzugsweise wenigstens 90 Gew.-%, des eingesetzten Topramezons auskristallisieren.

Die Kristallisation der Form I lässt sich durch Animpfen mit Impfkristallen der Form I fördern oder beschleunigen, beispielsweise indem vor oder während der Kristallisation Impfkristalle der Form I zugesetzt werden.

Sofern man bei der Kristallisation Impfkristalle zusetzt, beträgt ihre Menge typischerweise 0,001 bis 10 Gew.-%, häufig 0,005 bis 5 Gew.-%, insbesondere 0,01 bis 1 Gew.-% und speziell 0,05 bis 0,5 Gew.-%, bezogen auf das gelöste Topramezon.

Sofern man die Kristallisation in Gegenwart von Impfkristallen der Form I durchführt, werden diese vorzugsweise erst bei einer Temperatur zugegeben, bei der die Sättigungskonzentration des Topramezons in dem jeweiligen Lösungsmittel erreicht ist, d. h. bei oder unterhalb derjenigen Temperatur, bei der die gelöste Menge an Topramezon in dem jeweiligen Lösungsmittel eine gesättigte Lösung bildet. Die Temperaturabhängigkeit der Sättigungskonzentration in einem Lösungsmittel kann der Fachmann in Routineexperimenten ermitteln.

Alternativ kann man die Form I des Topramezons herstellen, indem man Topramezon in einem C₁-C₄-Alkanol oder C₂-C₄-Alkandiol oder in einem Gemisch aus C₁-C₄-Alkanol oder C₂-C₄-Alkandiol mit Wasser suspendiert und das suspendierte Material in der Suspension bewegt, z. B. durch Rühren. Das Bewegen erfolgt vorzugsweise über einen längeren Zeitraum, z. B. wenigstens 2 h, z. B. 2 h bis 6 d, insbesondere 4 h bis 72 h.

Sofern man ein Gemisch aus C₁-C₄-Alkanol oder C₂-C₄-Alkandiol mit Wasser verwendet, wird das Mengenverhältnis von Alkanol bzw. Alkandiol zu Wasser so gewählt, dass Topramezon nicht vollständig gelöst vorliegt. Das Volumenverhältnis Alkanol:Wasser bzw. Alkandiol:Wasser liegt vorzugsweise im Bereich von 1 : 20 bis 10 : 1.

Das Suspendieren des Topramezons erfolgt üblicherweise bei Temperaturen im Bereich von 20 bis 120 °C. Gegebenenfalls kann das Suspendieren bei erhöhter Temperatur durchgeführt werden, um die Umwandlung zu beschleunigen. Insbesondere wird man dann das Topramezon zumindest einen gewissen Zeitraum bei erhöhter Temperatur, insbesondere bei wenigstens 50 °C suspendieren, wobei die angewendete Temperatur naturgemäß den Siedepunkt des Lösungsmittels bzw. Lösungsmittel-WasserGemisch nicht überschreiten wird. Häufig erfolgt das Suspendieren zeitweilig bei Temperaturen im Bereich von 50 bis 140 °C. Vorzugsweise kühlt man vor dem Abtrennen der Mutterlauge die Suspension auf eine Temperatur unterhalb 30 °C, z. B. auf eine Temperatur im Bereich von 5 bis 30 °C ab und/oder engt die Suspension ein. Vorzugsweise werden die Bedingungen so gewählt, dass beim Abtrennen der Mutterlauge weniger als 20 Gew.-%, vorzugsweise weniger als 10 Gew.-%, des eingesetzten Topramezons in der Mutterlauge gelöst verbleiben.

Die Gewinnung der Form I aus dem Kristallisat, d. h. die Abtrennung der Form I von der Mutterlauge, gelingt durch übliche Techniken zur Trennung fester Bestandteile von Flüssigkeiten, z. B. durch Filtration, Zentrifugieren oder durch Dekantieren. In der Regel wird man den isolierten Feststoff waschen, z. B. mit dem zur Kristallisation verwendeten Lösungsmittel, mit Wasser oder mit einem Gemisch aus dem zur Kristallisation verwendeten organischen Lösungsmittel mit Wasser. Das Waschen kann in ein oder mehreren Schritten erfolgen, wobei man häufig im letzten Waschschritt mit Wasser wäscht. Das Waschen erfolgt typischerweise bei Temperaturen unterhalb 30 °C, häufig unterhalb 25 °C und insbesondere unterhalb 20 °C, um den Verlust an Wertprodukt möglichst gering zu halten. Anschließend kann man die erhaltene Form I trocknen und dann der Weiterverarbeitung zuführen. Häufig wird man jedoch den nach Waschen erhaltenen feuchten Wirkstoff, insbesondere einen wasserfeuchten Wirkstoff direkt der weiteren Verarbeitung zuführen.

Vorzugsweise setzt man zum Suspendieren ein Topramezon ein, das eine chemische Reinheit von wenigstens 85 %, häufig wenigstens 90 %, insbesondere wenigstens 95 %, aufweist, d. h. der Anteil organischer Verunreinigungen, die kein organisches Lösungsmittel sind, macht nicht mehr als 15 Gew.-%, häufig nicht mehr als 10 Gew.-% und insbesondere nicht mehr als 5 Gew.-%, bezogen auf das suspendiert vorliegende Topramezon, aus.

Durch die erfindungsgemäßen Verfahren wird die Form I mit einem Gehalt an Topramezon von in der Regel wenigstens 90 Gew.-%, häufig 94 Gew.-%, insbesondere wenigstens 96 Gew.-% erhalten. Der Anteil an Form I, bezogen auf die Gesamtmenge an Topramezon liegt typischerweise bei wenigstens 90 % und häufig wenigstens 95 % und speziell wenigstens 97 %.

Die Herstellung des als Ausgangsmaterial für die Herstellung der Form I verwendeten [3-(4,5-Dihydro-3-isoxazolyl)-2-methyl-4-(methylsulfonyl)phenyl]-(5-hydroxy-1-methyl-1H-pyrazol-4-yl)methanon kann nach den in WO 99/58509 (z. B. gemäß Beispiel 8 oder 9) beschriebenen Verfahren erfolgen, auf das hiermit in vollem Umfang Bezug genommen wird.

Im Rahmen der Untersuchungen zu kristallinen Modifikationen des Topramezons wurde auch eine weitere Form II identifiziert. Hierbei handelt es sich insbesondere um eine kristalline Form des Topramezons, die zu wenigstens 90 Gew.-%, insbesondere wenigstens 95 Gew.-% aus der kristallinen Form II besteht.

Die Form II kann mittels Röntgenpulverdiffraktometrie anhand ihres Beugungsdiagramms identifiziert werden. So zeigt ein bei 30 °C unter Verwendung von Cu-Kα-Strahlung (1,54178 Å) aufgenommenes Röntgenpulverdiffraktogramm wenigstens 5, häufig wenigstens 6 insbesondere wenigstens 7 und speziell alle der in der folgenden Tabelle 3 als 2θ-Werte, bzw. als Netzebenenabstände d, angegebenen Reflexe:

**Tabelle 3:**

| 2θ | d [Å] |
|---|---|
| 7,8 ± 0,2 | 11,29 ± 0,07 |
| 8,6 ± 0,2 | 10,33 ± 0,05 |
| 12,4 ± 0,2 | 7,10 ± 0,05 |
| 13,8 ± 0,2 | 6,40 ± 0,04 |
| 14,7 ± 0,2 | 6,01 ± 0,03 |
| 15,7 ± 0,2 | 5,65 ± 0,03 |
| 19,3 ± 0,2 | 4,59 ± 0,02 |
| 22,2 ± 0,2 | 4,00 ± 0,02 |
| 30,4 ± 0,2 | 3,69 ± 0,02 |

Untersuchungen an Einkristallen der Form II zeigen, dass die zugrunde liegende Kristallstruktur triklin ist. Die Elementarzelle weist die Raumgruppe P-1 auf. Die charakteristischen Daten der Kristallstruktur von Form II (bestimmt bei -173 °C) sind in der Tabelle 4 zusammengefasst.

**Tabelle 4: Kristallographische Eigenschaften der Form II**

| Parameter | Form II |
|---|---|
| Klasse | triklin |
| Raumgruppe | P-1 |
| a | 5,620(2) Å |
| b | 12,097(3) Å |
| c | 23,921 (5) Å |
| α | 94,63(1)° |
| β | 90,61(2)° |
| γ | 98.28(2)° |
| Volumen | 1603,7(6) Å³ |
| Z | 4 |
| Dichte (berechnet) | 1,51 g/cm³ |
| R¹ ; ωR² | 0,084, 0,129 |
| Wellenlänge | 1,54178 Å |

| | |
|---|---|
| a,b,c = Kantenlänge der Elementarzelle α,β,γ = Winkel der Elementarzelle Z = Anzahl der Moleküle in der Elementarzelle | |

Form II zeigt ein Thermogramm mit charakteristischem Schmelzpeak. Der Schmelzpunkt, bestimmt als Beginn des Schmelzpeaks (onset), liegt typischerweise im Bereich von 222 bis 223 °C. Die Schmelzwärme beträgt etwa 110 J/g. Die hier angegebenen Werte beziehen sich auf mittels Differentialkalorimetrie (Differential Scanning Calorimetry: DSC, offener Tiegel aus Aluminium, Aufheizrate 10 K/min) ermittelte Werte.

Die Herstellung der Form II des Topramezons gelingt durch Kristallisation aus einer Lösung von Topramezon in einem aromatischen Lösungsmittel oder einem C₅-C₈-Keton bei Temperaturen unterhalb 60 °C, insbesondere bei Temperaturen im Bereich von 10 bis 50 °C.

Beispiele für aromatische Lösungsmittel sind Benzol, Alkylbenzole wie Toluol, Xylole, Mesitylen, Cumol, etc., Chlorbenzol, 1,2-Dichlorbenzol, 1,3-Dichlorbenzol, 1,4-Dichlorbenzol und Dichlorbenzolgemische, Anisol und Acetophenon. Beispiele für C₅-C₈-Ketone sind acyclische Ketone mit 5 bis 8 Kohlenstoffatomen wie Diethylketon (3-Pentanon), Methylisobutylketon (4-Methylpentan-2-on) und cyclische Ketone mit 5 bis 8 C-Atomen wie Cyclopentanon, Cyclohexanon etc.

Hierzu stellt man in einem ersten Schritt i) eine Lösung des Topramezons in dem gewünschten Lösungsmittel bereit und bewirkt anschließend in einem zweiten Schritt ii) eine Kristallisation des Topramezons bei Temperaturen unterhalb 60 °C, insbesondere bei Temperaturen im Bereich von 10 bis 50 °C. Zum Lösen kann die Mischung von Topramezon und dem gewünschten Lösungsmittel auf Temperaturen oberhalb der genannten Temperaturen erhitzt werden. Wesentlich ist, dass bei einer Temperatur von 60 °C noch keine Kristallisation eingesetzt hat und dass die Kristallisation erst bei Temperaturen unterhalb 60 °C einsetzt.

Die Kristallisation der Form II des Topramezons kann in folgender Weise bewirkt werden, z. B.
- durch Abkühlen der Lösung, welche das Topramezon gelöst enthält auf eine Temperatur unterhalb 60 °C,
- durch Einengen der Lösung, welche das Topramezon gelöst enthält bei einer Temperatur unterhalb 60 °C, oder
- durch eine Kombination der vorgenannten Maßnahmen.

Vorzugsweise bewirkt man die Kristallisation durch Einengen bei Temperaturen in dem genannten Temperaturbereich.

Die Konzentration an Topramezon in der zur Kristallisation eingesetzten Lösung liegt häufig im Bereich von 10 bis 300 g/L.

Vorzugsweise enthält die zur Kristallisation der Form II eingesetzte Lösung Topramezon in einer Reinheit von wenigstens 85 %, häufig wenigstens 90 %, insbesondere wenigstens 95 %, d. h. der Anteil organischer Verunreinigungen, die kein organisches Lösungsmittel sind, macht nicht mehr als 15 Gew.-%, häufig nicht mehr als 10 Gew.-% und insbesondere nicht mehr als 5 Gew.-%, bezogen auf das in der Lösung gelöst vorliegende Topramezon aus.

Die zur Kristallisation eingesetzte Lösung ist vorzugsweise im Wesentlichen frei von anderen Lösungsmitteln als aromatische Lösungsmittel und C₅-C₈-Ketone. "Im Wesentlichen frei" bedeutet in diesem Zusammenhang, dass die Konzentration an Lösungsmitteln einschließlich Wasser, die von aromatischen Lösungsmittel oder einem C₅-C₈-Keton verschieden sind, in der Topramezon enthaltenden Lösung 10 Gew.-% und häufig 5 Gew.-%, bezogen auf die Gesamtmenge an Lösungsmittel, nicht überschreitet.

Zur Herstellung der Lösung kann grundsätzlich jede bekannte Form des Topramezons eingesetzt werden. Häufig wird man amorphes Topramezon oder eine Mischung verschiedener kristalliner Modifikationen oder ein Gemisch aus amorphem und kristallinem Topramezon einsetzen. Geeignet sind auch kristalline Formen des Topramezons sowie deren Mischungen, z. B. die zuvor beschriebene, erfindungsgemäße Form I und die ebenfalls hier beschriebene Form III sowie die Formen IV, V-S, VI-S und VII-S sowie Mischungen dieser Formen.

Die Kristallisation der Form II lässt sich durch Animpfen mit Impfkristallen der Form II fördern oder beschleunigen, beispielsweise indem vor oder während der Kristallisation Impfkristalle der Form II zugesetzt werden.

Sofern man bei der Kristallisation Impfkristalle zusetzt, beträgt ihre Menge typischerweise 0,001 bis 10 Gew.-%, häufig 0,005 bis 5 Gew.-%, insbesondere 0,01 bis 1 Gew.-% und speziell 0,05 bis 0,5 Gew.-%, bezogen auf das gelöste Topramezon. Sofern man die Kristallisation in Gegenwart von Impfkristallen der Form II durchführt, werden diese vorzugsweise erst bei einer Temperatur zugegeben, bei der die Sättigungskonzentration des Topramezons in dem jeweiligen Lösungsmittel erreicht ist, d. h. bei oder unterhalb derjenigen Temperatur, bei der die gelöste Menge an Topramezon in dem Lösungsmittel eine gesättigte Lösung bildet. Die Temperaturabhängigkeit der Sättigungskonzentration in einem Lösungsmittel kann der Fachmann in Routineexperimenten ermitteln.

Die Gewinnung der Form II aus dem Kristallisat, d. h. die Abtrennung der Form II von der Mutterlauge, gelingt durch übliche Techniken wie sie im Zusammenhang mit der Form I beschrieben werden.

Durch die Kristallisation wird die Form II mit einem Gehalt an Topramezon von in der Regel wenigsten 90 Gew.-%, häufig wenigstens 94 Gew.-%, insbesondere wenigstens 96 Gew.-% erhalten. Der Anteil an Form II, bezogen auf die Gesamtmenge an Topramezon liegt typischerweise bei wenigstens 90 %, häufig wenigstens 95 % und insbesondere wenigstens 97 %.

Im Rahmen der Untersuchungen zu kristallinen Modifikationen des Topramezons wurde auch eine weitere Form III identifiziert. Hierbei handelt es sich insbesondere um eine kristalline Form des Topramezons, die zu wenigstens 90 Gew.-%, insbesondere wenigstens 95 Gew.-% aus der kristallinen Form III besteht.

Die Form III kann mittels Röntgenpulverdiffraktometrie anhand ihres Beugungsdiagramms identifiziert werden. So zeigt ein bei 30 °C unter Verwendung von Cu-Kα-Strahlung (1,54178 Å) aufgenommenes Röntgenpulverdiffraktogramm wenigstens 3, häufig wenigstens 4 insbesondere wenigstens 5 und speziell alle der in der folgenden Tabelle 5 als 2θ-Werte, bzw. als Netzebenenabstände d, angegebenen Reflexe:

**Tabelle 5:**

| 2θ | d [Å] |
|---|---|
| 11,1 ± 0,2 | 8,00 ± 0,05 |
| 12,0 ± 0,2 | 7,36 ± 0,05 |
| 14,5 ± 0,2 | 6,09 ± 0,04 |
| 17,3 ± 0,2 | 5,11 ± 0,03 |
| 17,9 ± 0,2 | 4,94 ± 0,03 |
| 21,9 ± 0,2 | 4,06 ± 0,02 |
| 24,2 ± 0,2 | 3,67 ± 0,02 |

Form III zeigt ein Thermogramm mit charakteristischem Schmelzpeak. Der Schmelzpunkt, bestimmt als Beginn des Schmelzpeaks (onset), liegt typischerweise im Bereich von 223 bis 224 °C. Die Schmelzwärme beträgt etwa 109 J/g. Die hier angegebenen Werte beziehen sich auf mittels Differentialkalorimetrie (Differential Scanning Calorimetry: DSC, offener Tiegel aus Aluminium, Aufheizrate 10 K/min) ermittelte Werte.

Die Herstellung der Form III des Topramezons gelingt durch Kristallisation aus einer Lösung von Topramezon in einem aromatischen Lösungsmittel oder einem C₅-C₈-Keton bei Temperaturen oberhalb 60 °C, insbesondere bei Temperaturen im Bereich von 90 bis 140 °C.

Beispiele für aromatische Lösungsmittel sind Benzol, Alkylbenzole wie Toluol, Xylole, Mesitylen, Cumol, etc., Chlorbenzol, 1,2-Dichlorbenzol, 1,3-Dichlorbenzol, 1,4-Dichlorbenzol und Dichlorbenzolgemische, Anisol und Acetophenon. Beispiele für C₅-C₈-Ketone sind acyclische Ketone mit 5 bis 8 Kohlenstoffatomen wie Diethylketon (3-Pentanon), Methylisobutylketon (4-Methylpentan-2-on) und cyclische Ketone mit 5 bis 8 C-Atomen wie Cyclopentanon, Cyclohexanon etc.

Hierzu stellt man in einem ersten Schritt i) eine Lösung des Topramezons in dem gewünschten Lösungsmittel bereit und bewirkt anschließend in einem zweiten Schritt ii) eine Kristallisation des Topramezons bei Temperaturen oberhalb 80 °C, insbesondere bei Temperaturen im Bereich von 90 bis 150 °C. Zum Lösen wird man die Mischung von Topramezon und dem gewünschten Lösungsmittel auf Temperaturen oberhalb der genannten Temperaturen erhitzten und anschließend bei eine Temperatur in diesem Bereich eine Kristallisation bewirken. Wesentlich ist, dass die Kristallisation weitgehend oberhalb 80 °C abläuft.

Die Kristallisation der Form III des Topramezons kann in folgender Weise bewirkt werden, z. B.
- durch Abkühlen der Lösung, welche das Topramezon gelöst enthält, auf eine Temperatur unterhalb der zum Lösen angewendeten Temperatur jedoch oberhalb 80 °C und insbesondere im Bereich von 90 bis 150 °C,
- durch Einengen der Lösung, welche das Topramezon gelöst enthält, bei einer Temperatur oberhalb 80 °C und insbesondere im Bereich von 90 bis 150 °C, oder
- durch eine Kombination der vorgenannten Maßnahmen.

Vorzugsweise bewirkt man die Kristallisation durch Einengen bei Temperaturen in dem genannten Temperaturbereich.

Die Konzentration an Topramezon in der zur Kristallisation eingesetzten Lösung liegt häufig im Bereich von 10 bis 300 g/L.

Vorzugsweise enthält die zur Kristallisation der Form III eingesetzte Lösung Topramezon in einer Reinheit von wenigstens 85 %, häufig wenigstens 90 %, insbesondere wenigstens 95 %, d. h. der Anteil organischer Verunreinigungen, die kein organisches Lösungsmittel sind, macht nicht mehr als 15 Gew.-%, häufig nicht mehr als 10 Gew.-% und insbesondere nicht mehr als 5 Gew.-%, bezogen auf das in der Lösung gelöst vorliegende Topramezon aus.

Die zur Kristallisation eingesetzte Lösung ist vorzugsweise im Wesentlichen frei von anderen Lösungsmitteln als aromatische Lösungsmittel und C₅-C₈-Ketone. "Im Wesentlichen frei" bedeutet in diesem Zusammenhang, dass die Konzentration an Lösungsmitteln einschließlich Wasser, die von aromatischen Lösungsmittel oder einem C₅-C₈-Keton verschieden sind, in der Topramezon enthaltenden Lösung 10 Gew.-% und häufig 5 Gew.-%, bezogen auf die Gesamtmenge an Lösungsmittel, nicht überschreitet.

Zur Herstellung der Lösung kann grundsätzlich jede bekannte Form des Topramezons eingesetzt werden. Häufig wird man amorphes Topramezon oder eine Mischung verschiedener kristalliner Modifikationen oder ein Gemisch aus amorphem und kristallinem Topramezon einsetzen. Geeignet sind auch kristalline Formen des Topramezons sowie deren Mischungen, z. B. die zuvor beschriebene, erfindungsgemäße Form I und die ebenfalls hier beschriebene Form II sowie die Formen IV, V-S, VI-S, VII-S und VIII sowie Mischungen dieser Formen.

Die Kristallisation der Form III lässt sich durch Animpfen mit Impfkristallen der Form III all fördern oder beschleunigen, beispielsweise indem vor oder während der Kristallisation Impfkristalle der Form III zugesetzt werden.

Sofern man bei der Kristallisation Impfkristalle zusetzt, beträgt ihre Menge typischerweise 0,001 bis 10 Gew.-%, häufig 0,005 bis 5 Gew.-%, insbesondere 0,01 bis 1 Gew.-% und speziell 0,05 bis 0,5 Gew.-%, bezogen auf das gelöste Topramezon. Sofern man die Kristallisation in Gegenwart von Impfkristallen der Form III durchführt, werden diese vorzugsweise erst bei einer Temperatur zugegeben, bei der die Sättigungskonzentration des Topramezons in dem jeweiligen Lösungsmittel erreicht ist, d. h. bei oder unterhalb derjenigen Temperatur, bei der die gelöste Menge an Topramezon in dem Lösungsmittel eine gesättigte Lösung bildet. Die Temperaturabhängigkeit der Sättigungskonzentration in einem Lösungsmittel kann der Fachmann in Routineexperimenten ermitteln.

Die Gewinnung der Form III aus dem Kristallisat, d. h. die Abtrennung der Form III von der Mutterlauge, gelingt durch übliche Techniken wie sie im Zusammenhang mit der Form I beschrieben werden.

Durch die Kristallisation wird die Form III mit einem Gehalt an Topramezon von in der Regel wenigsten 90 Gew.-%, häufig wenigstens 94 Gew.-%, insbesondere wenigstens 96 Gew.-% erhalten. Der Anteil an Form III, bezogen auf die Gesamtmenge an Topramezon liegt typischerweise bei wenigstens 90 %, häufig wenigstens 95 % und insbesondere wenigstens 97 %.

Form III kann auch durch kontrolliertes Abkühlen einer Schmelze des Topramezons generiert werden. Hierzu geht man beispielsweise so vor, dass man eine Schmelze des Topramezons abkühlt und wieder auf eine Temperatur im Bereich von 110 °C bis 160 °C erwärmt. Hierbei setzt eine Kristallisation der Form III ein. Durch langsames Abkühlen erreicht man dann eine Vervollständigung der Kristallisation der Form III.

Im Zusammenhang mit der Untersuchung zur Kristallisation von Topramezon wurden zwei weitere Modifikationen IV und VIII sowie Solvate des Topramezons mit Toluol (Solvat V-S), mit Chlorbenzol (Solvat VI-S) und mit Dichlormethan (Solvat VII-S) aufgefunden. Im Vergleich zu Formulierungen, welche die Form I enthalten, weisen Formulierungen dieser Formen, z. B. wässrige Suspensionskonzentrate, ebenso wie Formulierungen der Formen II und III eine geringere Stabilität auf.

Die Form IV kann mittels Röntgenpulverdiffraktometrie anhand ihres Beugungsdiagramms identifiziert werden. So zeigt ein bei 30 °C unter Verwendung von Cu-Kα-Strahlung (1,54178 Å) aufgenommenes Röntgenpulverdiffraktogramm wenigstens 3, in der Regel wenigstens 5, häufig wenigstens 6 insbesondere wenigstens 7 und speziell alle der in der folgenden Tabelle 6 als 2θ-Werte, bzw. als Netzebenenabstände d, angegebenen Reflexe:

**Tabelle 6:**

| 2θ | d [Å] |
|---|---|
| 5,1 ± 0,2 | 17,34 ± 0,07 |
| 9,3 ± 0,2 | 9,41 ± 0,05 |
| 12,6 ± 0,2 | 7,00 ± 0,05 |
| 14,0 ± 0,2 | 6,34 ± 0,04 |
| 19,1 ± 0,2 | 4,63 ± 0,03 |
| 20,6 ± 0,2 | 4,31 ± 0,03 |
| 21,7 ± 0,2 | 4,10 ± 0,02 |
| 24,6 ± 0,2 | 3,61 ± 0,02 |
| 26,3 ± 0,2 | 3,38 ± 0,01 |

Untersuchungen an Einkristallen der Form IV zeigen, dass die zugrunde liegende Kristallstruktur monoklin ist. Die Elementarzelle weist die Raumgruppe P-1 auf. Die charakteristischen Daten der Kristallstruktur von Form IV (bestimmt bei -173 °C) sind in der Tabelle 7 zusammengefasst.

**Tabelle 7: Kristallographische Eigenschaften der Form IV**

| Parameter | Form IV |
|---|---|
| Klasse | Monoklin |
| Raumgruppe | P-1 |
| a | 13,9625(5) Å |
| b | 13,9080(5) Å |
| c | 17,796(5) Å |
| α | 90° |
| β | 107,729(5)° |
| γ | 90° |
| Volumen | 3292(2) Å³ |
| Z | 4 |
| Dichte (berechnet) | 1,47 g/cm³ |
| R¹ ; ωR² | 0,13, 0,44 |
| Wellenlänge | 1,54178 Å |

| | |
|---|---|
| a,b,c = Kantenlänge der Elementarzelle α,β,γ = Winkel der Elementarzelle Z = Anzahl der Moleküle in der Elementarzelle | |

Form IV zeigt ein Thermogramm mit charakteristischem Schmelzpeak. Der Schmelzpunkt, bestimmt als Beginn des Schmelzpeaks (onset), liegt typischerweise im Bereich von 224 bis 225 °C. Die Schmelzwärme beträgt etwa 114 bis 123 J/g. Die hier angegebenen Werte beziehen sich auf mittels Differentialkalorimetrie (Differential Scanning Calorimetry: DSC, offener Tiegel aus Aluminium, Aufheizrate 10 K/min) ermittelte Werte.

Die Herstellung der Form IV des Topramezons gelingt durch Kristallisation aus einer Lösung von Topramezon in einer Mischung aus Toluol oder Benzol mit Dichlormethan durch langsames Eindampfen der Lösung bei Temperaturen unterhalb 50 °C, insbesondere bei Temperaturen im Bereich von 10 bis 30 °C. In der Mischung liegt das Volumenverhältnis vorzugsweise im Bereich von 2 : 1 bis 1 : 4.

Kristallisation von Topramezon durch Eindampfen einer Lösung von Topramezon in einer Mischung aus Toluol mit Dichlormethan im Volumenverhältnis 2 : 1 bei Temperaturen unterhalb 30 °C, insbesondere bei Temperaturen im Bereich von 10 bis 30 °C liefert ein instabiles Solvat V-S, das 1 Mol Toluol pro Mol Topramezon enthält. Das Solvat gibt bereits bei Raumtemperatur das Lösungsmittel wieder ab und bildet dann ein Gemisch der Formen II und IV.

Untersuchungen an Einkristallen des Solvats V-S zeigen, dass die zugrunde liegende Kristallstruktur monoklin ist. Die Elementarzelle weist die Raumgruppe P2(1)/c auf. Die charakteristischen Daten der Kristallstruktur von Form V-S (bestimmt bei 20 °C) sind in der Tabelle 8 zusammengefasst.

**Tabelle 8: Kristallographische Eigenschaften des Solvats V-S.**

| Parameter | Form V-S |
|---|---|
| Klasse | monoklin |
| Raumgruppe | P2₁/c |
| a | 14,052(4) Å |
| b | 15,069(5) Å |
| c | 11,261(3) Å |
| α | 90 ° |
| β | 106,560(2)° |
| γ | 90 ° |
| Volumen | -2285,7(2) Å³ |
| Z | 4 |
| Dichte (berechnet) | 1,51 g/cm³ |
| R¹ ; ωR² | 0.084, 0.129 |
| Wellenlänge | 1,54178 Å |

| | |
|---|---|
| a,b,c = Kantenlänge der Elementarzelle α,β,γ = Winkel der Elementarzelle Z = Anzahl der Moleküle in der Elementarzelle | |

Kristallisation von Topramezon durch Eindampfen einer Lösung von Topramezon in einer Mischung aus Chlorbenzol mit Dichlormethan bei Temperaturen unterhalb 30 °C, insbesondere bei Temperaturen im Bereich von 10 bis 30 °C liefert ein instabiles Solvat VI-S, das 1 Mol Chlorbenzol pro Mol Topramezon enthält. Das Solvat gibt bereits bei Raumtemperatur das Lösungsmittel wieder ab und bildet dann die Form II.

Untersuchungen an Einkristallen des Solvats VI-S zeigen, dass die zugrunde liegende Kristallstruktur triklin ist. Die Elementarzelle weist die Raumgruppe P-1 auf. Die charakteristischen Daten der Kristallstruktur von Form VI-S (bestimmt bei -173 °C) sind in der Tabelle 9 zusammengefasst.

**Tabelle 9: Kristallographische Eigenschaften des Solvats VI-S.**

| Parameter | Form VI-S |
|---|---|
| Klasse | triklin |
| Raumgruppe | P-1 |
| a | 9,3084(8) Å |
| b | 10,2947(9) Å |
| c | 13,2043(9) Å |
| α | 67,473(4)° |
| β | 82,794(5)° |
| γ | 69,685(4) |
| Volumen | 1096,0(2) Å³ |
| Z | 2 |
| Dichte (berechnet) | 1,21 g/cm³ |
| R¹ ; ωR² | 0,062, 0,47 |
| Wellenlänge | 1,54178 Å |

| | |
|---|---|
| a,b,c = Kantenlänge der Elementarzelle α,β,γ = Winkel der Elementarzelle Z = Anzahl der Moleküle in der Elementarzelle | |

Kristallisation von Topramezon durch Eindampfen einer Lösung von Topramezon in Dichlormethan bei Temperaturen unterhalb 30 °C, insbesondere bei Temperaturen im Bereich von 10 bis 30 °C liefert ein instabiles Solvat VII-S, das Dichlormethan enthält. Das Solvat gibt bereits bei Raumtemperatur das Lösungsmittel wieder ab und bildet dann die Form I.

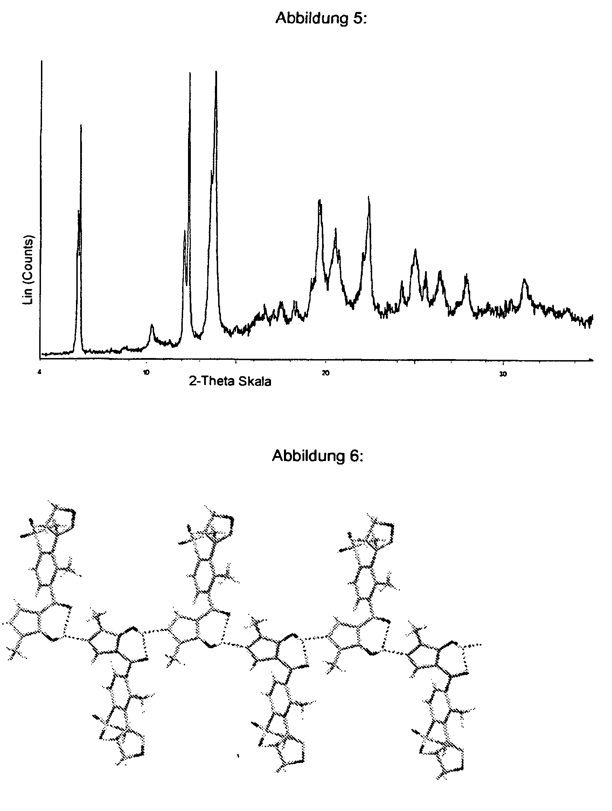

Das Solvat VII-S kann mittels Röntgenpulverdiffraktometrie anhand seines Beugungsdiagramms identifiziert werden. So zeigt Abbildung 5 ein bei 30 °C unter Verwendung von Cu-Kα-Strahlung (1,54178 Å) aufgenommenes Röntgenpulverdiffraktogramm dieser Probe.

Im Rahmen der Untersuchungen zu kristallinen Modifikationen des Topramezons wurde auch eine weitere Form VIII identifiziert. Hierbei handelt es sich insbesondere um eine kristalline Form des Topramezons, die zu wenigstens 90 Gew.-%, insbesondere wenigstens 95 Gew.-% aus der kristallinen Form VIII besteht.

Die Form VIII kann mittels Röntgenpulverdiffraktometrie anhand ihres Beugungsdiagramms identifiziert werden. So zeigt ein bei 30 °C unter Verwendung von Cu-Kα-Strahlung (1,54178 Å) aufgenommenes Röntgenpulverdiffraktogramm wenigstens 3, häufig wenigstens 4 insbesondere wenigstens 5 und speziell alle der in der folgenden Tabelle 10 als 2θ-Werte, bzw. als Netzebenenabstände d, angegebenen Reflexe:

**Tabelle 10:**

| 2θ | d [Å] |
|---|---|
| 8,3 ± 0,2 | 11,46 ± 0,07 |
| 16,0 ± 0,2 | 6,52 ± 0,05 |
| 21,4 ± 0,2 | 4,15 ± 0,03 |
| 3,9 ± 0,2 | 22,89 ± 0,02 |
| 25,0 ± 0,2 | 3,56 ± 0,02 |
| 30,6 ± 0,2 | 2,91 ± 0,02 |

Form VIII zeigt ein Thermogramm mit charakteristischem Schmelzpeak. Der Schmelzpunkt, bestimmt als Beginn des Schmelzpeaks (onset), liegt typischerweise im Bereich von 223 bis 224 °C. Die Schmelzwärme beträgt etwa 115 J/g. Die hier angegebenen Werte beziehen sich auf mittels Differentialkalorimetrie (Differential Scanning Calorimetry: DSC, offener Tiegel aus Aluminium, Aufheizrate 10 K/min) ermittelte Werte.

Die Form I des Topramezons eignet sich ebenso wie das bekannte amorphe Topramezon und andere feste Formen des Topramezons als Herbizid, ist jedoch diesen bezüglich seiner Handhabbarkeit und Formulierbarkeit überlegen. Die Erfindung betrifft daher auch Pflanzenschutzmittel, enthaltend die kristalline Form I und für die Formulierung von Pflanzenschutzmitteln übliche Hilfsmittel, insbesondere Pflanzenschutzmittel in Form von wässrigen Suspensionskonzentraten (so genannte SC's) oder nicht-wässrigen Suspensionskonzentraten (so genannte OD's (oil dispersion bzw. oil-based suspension)), sowie Pflanzenschutzmittel in Form von in Wasser dispergierbaren Pulvern (so genannte WP's) und Granulaten (so genannte WG's). Die Erfindung betrifft auch ein Verfahren zur Bekämpfung von unerwünschtem Pflanzenwuchs, das dadurch gekennzeichnet ist, dass man die Form I des Topramezons, vorzugsweise als geeignete Wirkstoffaufbereitung auf Pflanzen, deren Lebensraum und/oder auf Samen einwirken lässt.

Die Pflanzenschutzmittel, welche Topramezon in der Form I enthalten, bekämpfen Pflanzenwuchs, insbesondere monokotyle Unkrautarten wie Avena, Lolium, Alopecurus, Phalaris, Echinochloa, Digitaria, Setaria, Cyperusarten, Agropyron, Cynodon, Imperato und Sorghum, sowie dikotyle Unkrautarten wie Galium, Viola, Veronica, Lamium, Stellaria, Amaranthus, Sinapsis, Ipomoea, Matricaria, Abutilon, Sida, Convolvolus, Cirsium, Rumex und Artemisia auf Nichtkulturflächen sehr gut, besonders bei hohen Aufwandmengen. In Kulturen wie Weizen, Gerste, Roggen, Reis, Mais, Zuckerrübe, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode kann die Form I des Topramezons bzw. die sie enthaltenden Pflanzenschutzmittel noch in einer weiteren Zahl von Kulturpflanzen zur Beseitigung unerwünschter Pflanzen eingesetzt werden, In Betracht kommen beispielsweise folgende Kulturen:
Allium cepa, Ananas comosus, Arachis hypogaea, Asparagus officinalis, Avena sativa Beta vulgaris spec. altissima, Beta vulgaris spec. rapa, Brassica napus var. napus, Brassica napus var. napobrassica, Brassica rapa var. silvestris, Brassica oleracea, Brassica nigra, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa , Phaseolus lunatus, Phaseolus vulgaris, Picea abies, Pinus spec., Pistacia vera, Pisum sativum, Prunus armeniaca, Prunus avium, Prunus cerasus, Prunus dulcis, Prunus domestica, Prunus persica, Pyrus communis, Ribes sylvestre, Ricinus communis, Saccharum officinarum, Secale cereale, Sinapis alba, Solanum tuberosum, Sorghum bicolor (s. vulgare), Theobroma cacao, Trifolium pratense, Triticale, Triticum aestivum, Triticum durum, Vicia faba, Vitis vinifera und Zea mays.

Darüber hinaus kann Topramezon in der Form I bzw. können die sie enthaltenden Pflanzenschutzmittel auch in Kulturen, die durch Züchtung einschließlich gentechnischer Methoden gegen die Wirkung von Herbiziden tolerant sind, verwendet werden.

Weiterhin kann Topramezon in der Form I bzw. können die sie enthaltenden Pflanzenschutzmittel auch in Kulturen, die durch Züchtung einschließlich gentechnischer Methoden gegen Insekten- oder Pilzbefall tolerant sind, verwendet werden.

Die Form I des Topramezons eignet sich ebenso wie das bekannte amorphe Topramezon zur Defoliation und Desikkation von Pflanzenteilen, wofür Kulturpflanzen wie Baumwolle, Kartoffel, Raps, Sonnenblume, Sojabohne oder Ackerbohnen, insbesondere Baumwolle, in Betracht kommen. Diesbezüglich betreffen Ausführungsformen der Erfindung auch Mittel zur Desikkation und /oder Defoliation von Pflanzen, Verfahren zur Herstellung dieser Mittel und Verfahren zur Desikkation und/oder Defoliation von Pflanzen unter Verwendung der Form I des Topramezons.

Als Desikkant eignet sich die Form I des Topramezons insbesondere zur Austrocknung der oberirdischen Teile von Kulturpflanzen wie Kartoffel, Raps, Sonnenblume und Sojabohne aber auch Getreide. Damit wird ein vollständig mechanisches Beernten dieser wichtigen Kulturpflanzen ermöglicht.

Von wirtschaftlichem Interesse ist ferner die Emteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, d. h., die Förderung der Ausbildung von Trenngewebe zwischen Frucht- oder Blatt- und Sprossteil der Pflanzen ist auch für ein gut kontrollierbares Entblättern von Nutzpflanzen, insbesondere Baumwolle, wesentlich.

Außerdem führt die Verkürzung des Zeitintervalls, in dem die einzelnen Baumwollpflanzen reif werden, zu einer erhöhten Faserqualität nach der Ernte.

Topramezon in der Form I bzw. die sie enthaltenden Pflanzenschutzmittel können beispielsweise in Form von Pulvern, Suspensionen, auch hochprozentigen wässrigen, öligen oder sonstigen Suspensionen, Ölsuspensionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die erfindungsgemäßen Pflanzenschutzmittel enthalten Topramezon in der Form I, d. h. in einer Reinheit von wenigstens 90 Gew.-%, sowie Hilfsmittel und/oder Trägerstoffe, wie sie für die Formulierung von Pflanzenschutzmitteln üblich sind. Die Menge an Wirkstoff, d. h. die Gesamtmenge an Topramezon sowie an gegebenenfalls weiteren Wirkstoffen, liegt in derartigen Pflanzenschutzmitteln üblicherweise im Bereich von 1 bis 98 Gew.-%, insbesondere im Bereich von 10 bis 95 Gew.-%, bezogen auf das Gesamtgewicht des Pflanzenschutzmittels.

Als Trägerstoffe kommen grundsätzlich alle festen und flüssigen Substanzen in Betracht, die üblicherweise in Pflanzenschutzmitteln, insbesondere in Herbizid-Formulierungen als Trägerstoffe zum Einsatz kommen.

Feste Trägerstoffe sind z. B. Mineralerden, wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nussschalenmehl, Cellulosepulver und andere feste Trägerstoffe.

Flüssige Trägerstoffe sind neben Wasser auch organische Flüssigkeiten, z. B. Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z. B. Paraffine, Tetrahydronaphthalin, alkylierte Naphthaline und deren Derivate, alkylierte Benzole und deren Derivate, einschließlich aromatische und nicht-aromatische Kohlenwasserstoffgemische, z. B. die unter den Handelsbezeichnungen Exxsol und Solvesso vertriebenen Produkte, Alkohole wie Propanol, Butanol und Cyclohexanol, Ketone wie Cyclohexanon, stark polare aprotische Lösungsmittel, z. B. Amide wie N-Methylpyrrolidon.

Als Trägerstoffe kommen auch die für Topramezon typischerweise eingesetzten Adjuvantien in Betracht, die alleine oder in Kombination mit den vorgenannten Trägerstoffen eingesetzt werden und die typischerweise ausgewählt sind unter Penetratoren, Spreitmitteln und Haftvermittlern. Hierbei handelt es sich um Substanzen, die üblicherweise eine oberflächenaktive Wirkung aufweisen. Beispiele für Adjuvantien sind insbesondere Fettsäuremethylester, Fettsäuren, ethoxilierte Fettalkohole, Ethylenoxid-Propylenoxid-Blockcopolymere, Phosphat- und Sulfat-Ester ethoxilierter Fettalkohole, ethoxilierte Sorbitan-Fettsäureester, ethoxilierte Mono-, Di- und Triglyceride und der gleichen sowie Mischungen dieser Produkte, z. B. die unter den Handelsbezeichnungen Dash (BASF), Tween 20 (Uniquema), Hasten (Wilbur-Ellis), Break-Thru (Degussa-Goldschmidt) bekannten Produkte sowie die Lutensol-, Pluronic und Plurafac-Typen der BASF Aktiengesellschaft.

Typische Hilfsmittel umfassen oberflächenaktive Substanzen, insbesondere die in Pflanzenschutzmitteln üblicherweise eingesetzten Netzmittel, Emulgatoren und Dispergier(hilfs)mittel, weiterhin die Viskosität verändernde Additive (Verdicker, Andicker), Antischaummittel, Frostschutzmittel, Mittel zur Einstellung des pH-Wertes, Stabilisatoren, Anticaking-Mittel und Biozide (Konservierungsmittel).

Als oberflächenaktive Substanzen kommen vorzugsweise anionische und nichtionische Tenside in Betracht. Geeignete oberflächenaktive Substanzen sind auch Schutzkolloide.

Die Menge an oberflächenaktiven Substanzen wird in der Regel 0,1 bis 60 Gew.-%, insbesondere 0,5 bis 50 Gew.-%, bzw. bezogen auf das Gesamtgewicht der erfindungsgemäßen Pflanzenschutzmittel, bzw. 0,5 bis 200 Gew.-%, bezogen auf die Gesamtmenge an festen Wirkstoffen in der Formulierung, betragen. Vorzugsweise umfassen die oberflächenaktiven Substanzen wenigstens eine anionische oberflächenaktive Substanz und wenigstens eine nichtionische oberflächenaktive Substanz, wobei das Mengeverhältnis von anionischer zu nichtionischer oberflächenaktiver Substanz typischerweise im Bereich von 50 : 1 bis 1 : 50 liegt.

Zu den Beispielen anionischer Tenside zählen Alkylarylsulfonate, aromatische Sulfonate, z. B. Ligninsulfonate (Borresperse-Typen, Borregaard), Kraftlignine (Reax-Typen der MeadWestvaco), Oxylignine (Vanillex-Typen der Nippon Paper), Phenylsulfonate, Napthalinsulfonate (Morwet-Typen, Akzo Nobel), Dibutylnaphthalinsulfonate (Nekal-Typen, BASF), Alkylsulfate, insbesondere Fettalkoholsulfate, Laurylsulfate, sowie sulfatierte Hexadeca-, Heptadeca- und Octadecanole, Alkylsulfonate, Alkylethersulfate, insbesondere Fettalkohol(poly)glykolethersulfate, Alkylarylethersulfate, Alkylpolyglykoletherphosphate, Polyarylphenyletherphosphate, Alkylsulfosuccinate, Olefinsulfonate, Paraffinsulfonate, Petroleumsulfonate, Tauride, Sarkoside, Fettsäuren, Alkylnaphthalinsulfonsäuren, Naphthalinsulfonsäuren, Ligninsulfonsäuren, Polykondensationsprodukte von Fettsäuren, Kondensationsprodukte sulfonierter Naphthaline mit Formaldehyd, Kondensationsprodukte sulfonierter Naphthaline mit Formaldehyd und Phenol und gegebenenfalls Harnstoff sowie Kondensationsprodukte aus Phenolsulfonsäure, Formaldehyd und Harnstoff, Lignin-Sulfit-Ablauge, Alkylphosphate, Alkylarylphosphate, z. B. Tristyrylphosphate, sowie Polycarboxylate wie z. B. Polyacrylate, Maleinsäureanhydrid/Olefin-Copolymere (z. B. Sokalan^{®} CP9, BASF), einschließlich der Alkali-, Erdalkali-, Ammonium- und Amin-Salze der vorgenannten Substanzen. Bevorzugte anionische oberflächenaktive Substanzen sind solche, die wenigstens eine Sulfonatgruppe tragen und insbesondere deren Alkalimetall- und deren Ammoniumsalze.

Beispiele für nichtionische oberflächenaktiver Substanzen sind Alkylphenolalkoxylate, insbesondere Ethoxliate und Ethoxylat-co-propoxylate von Octylphenol, Isooctylphenol, Nonylphenol und Tributylphenol, Di- und Tristyrylphenol-Alkoxylate, Alkoholalkoxylate, insbesondere Fettalkohol-ethoxylate und Fettalkohol-ethoxylat-co-propoxylate, z. B. alkoxiliertes Isotridecanol, Fettaminalkoxylate, Polyoxyethylenglycerolfettsäureester, Rizinusölalkoxylate, Fettsäurealkoxylate, Fettsäureamidalkoxylate, Fettsäurepolydiethanolamide, Lanolinethoxylate, Fettsäurepolyglykolester, Isotridecylalkohol, ethoxilierte Fettsäureamide, ethoxilierte Fettsäureester, Alkylpolyglykoside, ethoxilierte Alkylpolyglykoside, Sorbitanfettsäureester, ethoxilierte Sorbitanfettsäureester, Glycerolfettsäureester, niedermolekulare Polyalkylenoxide wie Polyethylenglykol, Polypropylenoxid, Polyethylenoxid-co-Polypropylenoxid-Di- und Triblockcopolymere, und deren Gemische. Bevorzugte nichtionische oberflächenaktive Substanzen sind Fettalkohol-ethoxylate, Alkylpolyglykoside, Glycerolfettsäureester, Rizinusölethoxylate, Fettsäureethoxylate, Fettsäureamidethoxylate, Lanolinethoxylate, Fettsäurepolyglykolester, Ethylenoxid-Propylenoxid-Blockcopolymere und deren Mischungen.

Schutzkolloide sind typischerweise wasserlösliche, amphiphile Polymere, die im Unterschied zu den vorgenannten Tensiden typischerweise Molekulargewichte oberhalb 2000 Dalton aufweisen (Zahlenmittel). Beispiele hierfür sind Proteine und denaturierte Proteine wie Casein, Polysaccharide wie wasserlösliche Stärkederivate und Cellulosederivate, hydrophob modifizierte Stärken und Cellulosen, z. B. Methylcellulose, weiterhin Polycarboxylate wie Polyacrylsäure, Acrylsäurecopolymere und MaleinsäureCopolymere (BASF Sokalan-Typen), Polyvinylalkohol (Mowiol-Typen der Clariant), Polyalkoxylate, Polyvinylpyrrolidon, Vinylpyrrolidon-Copolymere, Polyvinylamine, Polyethylenimine (Lupasol-Typen der BASF), und höhermolekulare Polyalkylenoxide wie Polyethylenglykol, Polypropylenoxide, Polyethylenoxid-co-Polypropylenoxid-Di- und Triblockcopolymere.

Die erfindungsgemäßen Pflanzenschutzmittel können auch ein oder mehrere, die Viskosität verändernde Additive (Andicker) enthalten. Hierunter versteht man insbesondere Substanzen und Substanzgemische, die der Formulierung ein modifiziertes Fließverhalten verleihen, z. B. eine hohe Viskosität im Ruhezustand und niedrige Viskosität im bewegten Zustand. Die Art des Andickers richtet sich nach der Art der Formulierung. Als Beispiele für Andicker sind zu nennen: anorganische Substanzen, z. B. Schichtsilikate und organisch modifizierte Schichtsilikate wie Bentonite oder Attapulgite (z. B. Attaclay^{®} Firma Engelhardt), und organische Substanzen wie Polysaccharide und Heteropolysaccharide wie Xanthan Gum^{®} (Kelzan^{®} der Fa. Kelco), Rhodopol^{®} 23 (Rhone Poulenc) oder Veegum^{®} (Firma R.T. Vanderbilt). Die Menge der die Viskosität verändernden Additive beträgt häufig 0,1 bis 5 Gew.-%, bezogen auf das Gesamtgewicht des Pflanzenschutzmittels.

Beispiele für Antischaummittel sind die für diesen Zweck bekannte Silikonemulsionen (Silikon^{®} SRE, Firma Wacker oder Rhodorsil^{®} der Firma Rhodia), langkettige Alkohole, Fettsäuren und deren Salze, Entschäumer vom Typ wässriger Wachsdispersionen, feste Entschäumer (sog. Compounds), fluororganische Verbindungen und deren Gemische. Die Menge an Antischaummittel beträgt typischerweise 0,1 bis 1 Gew.-%, bezogen auf das Gesamtgewicht des Pflanzenschutzmittels.

Den erfindungsgemäßen Pflanzenschutzmitteln kann man zur Stabilisierung auch Konservierungsmittel zusetzen. Geeignete Konservierungsmittel sind solche auf Basis von Isothiazolonen, beispielsweise Proxel^{®} der Fa. ICI oder Acticide^{®} RS der Fa. Thor Chemie oder Kathon^{®} MK der Firma Rohm & Haas. Die Menge an Konservierungsmittel beträgt typischerweise 0,05 bis 0,5 Gew.-%, bezogen auf das Gesamtgewicht des SC's.

Wässrige Pflanzenschutzmittel, d. h. solche mit einem wässrigen Träger enthalten häufig Frostschutzmittel. Geeignete Frostschutzmittel sind flüssige Polyole, z. B. Ethylenglycol, Propylenglycol oder Glycerin sowie Harnstoff. Die Menge an Frostschutzmitteln beträgt in der Regel 1 bis 20 Gew.-%, insbesondere 5 bis 10 Gew.-%, bezogen auf das Gesamtgewicht des wässrigen Pflanzenschutzmittels.

Sofern die Pflanzenschutzmittel, enthaltend die kristalline Modifikation I, II oder III zur Saatgutbehandlung eingesetzt werden, können sie weitere übliche Bestandteile enthalten, wie sie bei der Saatgutbehandlung, z. B. bei Beizen oder Coaten eingesetzt werden. Hierzu zählen neben den vorgenannten Bestandteilen insbesondere Farbmittel, Kleber, Füllstoffe und Weichmacher.

Als Farbmittel kommen alle die für derartige Zwecke üblichen Farbstoffe und Pigmente in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C. I. Pigment Red 112 und C. I. Solvent Red 1, Pigment Blue 15:4, Pigment Blue 15:3, Pigment Blue 15:2, Pigment Blue 15:1, Pigment Blue 80, Pigment Yellow 1, Pigment Yellow 13, Pigment Red 48:2, Pigment Red 48:1, Pigment Red 57:1, Pigment Red 53:1, Pigment Orange 43, Pigment Orange 34, Pigment Orange 5, Pigment Green 36, Pigment Green 7, Pigment White 6, Pigment Brown 25, Basic Violet 10, Basic Violet 49, Acid Red 51, Acid Red 52, Acid Red 14, Acid Blue 9, Acid Yellow 23, Basic Red 10, Basic Red 108 bekannten Farbstoffe und Pigmente. Die Menge an Farbmittel wird üblicherweise nicht mehr als 20 Gew.-% der Formulierung ausmachen und liegt vorzugsweise im Bereich von 0,1 bis 15 Gew.-%, bezogen auf das Gesamtgewicht der Formulierung.

Als Kleber kommen alle üblichen in Beizmitteln einsetzbaren Bindemittel in Frage. Beispiele für geeignete Bindemittel umfassen thermoplastische Polymere wie Polyvinylpyrrolidon, Polyvinylacetat, Polyvinylalkohol und Tylose weiterhin Polyacrylate, Polymethacrylate, Polybutene, Polyisobutene, Polystyrol, Polyethylenamine, Polyethylenamide, die vorgenannten Schutzkolloide, Polyester, Polyetherester, Polyanhydride, Polyesterurethane, Polyesteramide, thermoplastische Polysaccharide, z. B. Cellulosederivate wie Celluloseester, Celluloseether, Celluloseetherester, einschließlich Methylcellulose, Ethylcellullose, Hydroxymethylcellulose, Carboxymethylcellulose, Hydroxypropylcellulose und Stärkederivate und modifizierte Stärken, Dextrine, Maltodextrine, Alginate und Chitosane, weiterhin Fette, Öle, Proteine, einschließlich Casein, Gelatine und Zein, Gummi-Arabicum, Schellack. Vorzugsweise sind die Kleber pflanzenverträglich, d. h. sie weisen keine oder keinen nennenswerten phytotoxischen Wirkungen auf. Vorzugsweise sind die Kleber biologisch abbaubar. Vorzugsweise ist der Kleber so gewählt, dass er als Matrix für die aktiven Bestandteile der Formulierung wirkt. Die Menge an Kleber wird üblicherweise nicht mehr als 40 Gew.-% der Formulierung ausmachen und liegt vorzugsweise im Bereich von 1 bis 40 Gew.-% und insbesondere im Bereich von 5 bis 30 Gew.-%, bezogen auf das Gesamtgewicht der Formulierung.

Neben dem Kleber kann die Formulierung für die Saatgutbehandlung auch inerte Füllstoffe enthalten. Beispiele hierfür sind die vorgenannten festen Trägermaterialien, insbesondere feinteilige anorganische Materialien wie Tone, Kreide, Bentonit, Kaolin, Talkum, Perlit, Mica, Kieselgel, Diatomeenerde, Quarzpulver, Montmorillonit, aber auch feinteilige organische Materialen, wie Holzmehl, Getreidemehl, Aktivkohle und dergleichen. Die Menge an Füllstoff wird vorzugsweise so gewählt, dass die Gesamtmenge an Füllstoff 70 Gew.-%, bezogen auf das Gesamtgewicht aller nicht flüchtigen Bestandteile der Formulierung nicht überschreitet. Häufig liegt die Menge an Füllstoff im Bereich von 1 bis 50 Gew.-%, bezogen auf das Gesamtgewicht aller nicht flüchtigen Bestandteile der Formulierung.

Daneben kann die Formulierung für die Saatgutbehandlung noch einen Weichmacher enthalten, der die Flexibilität der Beschichtung erhöht. Beispiele für Weichmacher sind oligomere Polyalkylenglykole, Glycerin, Dialkylphthalate, Alkylbenzylphthalate, Glykolbenzoate und vergleichbare Verbindungen. Die Menge an Weichmacher in der Beschichtung liegt häufig im Bereich von 0,1 bis 20 Gew.-%, bezogen auf das Gesamtgewicht aller nicht flüchtigen Bestandteile der Formulierung.

Eine bevorzugte Ausführungsform der Erfindung betrifft flüssige Formulierungen der Form I. Diese weisen neben der festen Wirkstoffphase wenigstens eine flüssige Phase auf, in der Topramezon in der Form I in Form dispergierter feiner Partikel vorliegt. Als flüssige Phase kommen grundsätzlich Wasser sowie solche organischen Lösungsmittel in Betracht, in denen die Form I nur eine geringe oder keine Löslichkeit besitzt, z. B. solche, in denen die Löslichkeit der Form I bei 25 °C und 1013 mbar nicht mehr als 1 Gew.-%, insbesondere nicht mehr als 0,1 Gew.% und speziell nicht mehr als 0,01 Gew.-% beträgt.

Gemäß einer ersten bevorzugten Ausführungsform handelt es sich bei der flüssigen Phase um Wasser und wässrige Lösungsmittel, d. h. Lösungsmittelgemische, die neben Wasser noch bis zu 20 Gew.-%, vorzugsweise jedoch nicht mehr als 10 Gew.-%, bezogen auf die Gesamtmenge an Wasser und Lösungsmittel, eines oder mehrere mit Wasser mischbare organische Lösungsmittel, z. B. mit Wasser mischbare Ether wie Tetrahydrofuran, Methylglykol, Methyldiglykol, Alkanole wie Isopropanol oder Polyole wie Glykol, Glycerin, Diethylenglykol, Propylenglykol und dergleichen, enthalten. Derartige Formulierungen werden im Folgenden auch als Suspensionskonzentrate (SC's) bezeichnet.

Derartige Suspensionskonzentrate enthalten Topramezon als Form I, als Form II oder als Form III in einer feinteiligen partikulären Form, wobei die Partikel der Form I, Form II bzw. Form III in einer wässrigen Phase suspendiert vorliegen. Die Größe der Wirkstoffpartikel, d. h. die Größe, welche 90 Gew.-% der Wirkstoffpartikel nicht überschreiten, liegt dabei typischerweise unterhalb 30 µm, insbesondere unterhalb 20 µm. Vorteilhafterweise weisen in den erfindungsgemäßen SC's wenigstens 40 Gew.-%, insbesondere wenigstens 60 Gew.-% und speziell wenigstens 80 Gew.-% der Teilchen Durchmesser unterhalb 2 µm auf.

Die Menge an Wirkstoff, d. h. die Gesamtmenge an Topramezon sowie an gegebenenfalls weiteren Wirkstoffen, liegt in derartigen SC's üblicherweise im Bereich von 10 bis 70 Gew.-%, insbesondere im Bereich von 20 bis 50 Gew.-%, bezogen auf das Gesamtgewicht des Suspensionskonzentrats.

Wässrige Suspensionskonzentrate enthalten neben dem Wirkstoff typischerweise oberflächenaktive Substanzen, sowie gegebenenfalls Antischaummittel, Verdicker (= Andicker), Frostschutzmittel, Stabilisatoren (Biozide), Mittel zur Einstellung des pH-Wertes und Anticaking-Mittel.

Wässrige Formulierungen weisen typischerweise einen pH-Wert < 8, insbesondere < 6 auf.

Als oberflächenaktive Substanzen kommen die zuvor genannten oberflächenaktiven Substanzen in Betracht. Vorzugsweise enthalten die erfindungsgemäßen wässrigen Pflanzenschutzmittel wenigstens eines der zuvor genannten anionischen Tenside und gegebenenfalls ein oder mehrere nichtionische Tenside, gegebenenfalls in Kombination mit einem Schutzkolloid. Die Menge an oberflächenaktiven Substanzen wird in der Regel 1 bis 50 Gew.-%, insbesondere 2 bis 40 Gew.-%, bezogen auf das Gesamtgewicht der erfindungsgemäßen wässrigen SC's betragen. Vorzugsweise umfassen die oberflächenaktiven Substanzen wenigstens eine anionische oberflächenaktive Substanz und wenigstens eine nichtionische oberflächenaktive Substanz, wobei das Mengeverhältnis von anionischer zu nichtionischer oberflächenaktiver Substanz typischerweise im Bereich von 50 : 1 bis 1 : 50 liegt.

Bezüglich der Art und Menge der Antischaummittel, Verdicker, Frostschutzmittel und Biozide gilt das zuvor Gesagte.

Gegebenenfalls können die erfindungsgemäßen wässrigen SC's Puffer zur pH-Wert Regulation enthalten. Beispiele für Puffer sind Alkalisalze schwacher anorganischer oder organischer Säuren wie z. B. Phosphorsäure, Borsäure, Essigsäure, Propionsäure, Citronensäure, Fumarsäure, Weinsäure, Oxalsäure und Bernsteinsäure.

Gemäß einer weiteren bevorzugten Ausführungsform handelt es sich bei der flüssigen Phase um nicht-wässrige organische Lösungsmittel, in denen die Löslichkeit der Form I des Topramezons bei 25 °C und 1013 mbar nicht mehr als 1 Gew.-%, insbesondere nicht mehr als 0,1 Gew.-% und speziell nicht mehr als 0,01 Gew.-% beträgt. Hierzu zählen insbesondere aliphatische und cycloaliphatische Kohlenwasserstoffe sowie Öle, insbesondere solche pflanzlichen Ursprungs, weiterhin C₁-C₄-Alkylester von gesättigten oder ungesättigten Fettsäuren oder Fettsäuregemischen, insbesondere die Methylester, z. B. Ölsäuremethylester, Stearinsäuremethylester, Rapsölmethylester, aber auch paraffinische Mineralöle und dergleichen. Dementsprechend betrifft die vorliegende Erfindung auch Mittel für den Pflanzenschutz in Form eines nicht-wässrigen Suspensionskonzentrats, das im Folgenden auch als OD (Oil-dispersion) bezeichnet wird. Derartige OD's enthalten die Form I des Topramezons in einer feinteiligen partikulären Form, wobei die Partikel der Form I in einer nicht-wässrigen Phase suspendiert vorliegen. Die Größe der Wirkstoffpartikel, d. h. die Größe, welche 90 Gew.-% der Wirkstoffpartikel nicht überschreiten, liegt dabei typischerweise unterhalb 30 µm, insbesondere unterhalb 20 µm. Vorteilhafterweise weisen in den nicht-wässrigen Suspensionskonzentrate wenigstens 40 Gew.-%, insbesondere wenigstens 60 Gew.-% und speziell wenigstens 80 Gew.-% der Teilchen Durchmesser unterhalb 2 µm auf.

Die Menge an Wirkstoff, d. h. die Gesamtmenge an Topramezon sowie an gegebenenfalls weiteren Wirkstoffen, liegt in derartigen OD's üblicherweise im Bereich von 10 bis 70 Gew.-%, insbesondere im Bereich von 20 bis 50 Gew.-%, bezogen auf das Gesamtgewicht des nicht-wässrigen Suspensionskonzentrats.

Nicht-wässrige Suspensionskonzentrate enthalten neben dem Wirkstoff und dem flüssigen Trägerstoff typischerweise oberflächenaktive Substanzen, sowie gegebenenfalls Antischaummittel, Mittel zur Modifizierung der Rheologie sowie Stabilisatoren (Biozide).

Als oberflächenaktive Substanzen kommen vorzugsweise die zuvor genannten anionischen und nichtionischen Tenside in Betracht. Die Menge an oberflächenaktiven Substanzen wird in der Regel 1 bis 40 Gew.-%, insbesondere 2 bis 30 Gew.-%, bezogen auf das Gesamtgewicht der erfindungsgemäßen nicht-wässrigen SC's betragen. Vorzugsweise umfassen die oberflächenaktiven Substanzen wenigstens eine anionische oberflächenaktive Substanz und wenigstens eine nichtionische oberflächenaktive Substanz, wobei das Mengeverhältnis von anionischer zu nichtionischer oberflächenaktiver Substanz typischerweise im Bereich von 20 : 1 bis 1 : 20 liegt.

Die erfindungsgemäße Form I des Topramezons kann auch als festes Pflanzenschutzmittel formuliert werden. Hierzu zählen Pulver-, Streu- und Stäubemittel aber auch wasserdispergierbare Pulver und Granulate, z. B. Umhüllungs-, Imprägnierungsund Homogengranulate. Derartige Formulierungen können durch Mischen oder gemeinsames Vermahlen der Form I des Topramezons mit einem festen Trägerstoff und gegebenenfalls weiteren Hilfsmitteln, insbesondere oberflächenaktiven Substanzen, hergestellt werden. Granulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nussschalenmehl, Cellulosepulver oder andere feste Trägerstoffe. Feste Formulierungen können auch durch Sprühtrocknung, gegebenenfalls in Gegenwart von polymeren oder anorganischen Trocknungshilfsmitteln, und gegebenenfalls in Gegenwart fester Trägerstoffe hergestellt werden. Für die Herstellung fester Formulierungen des Topramezons der Form I sind auch Extrusionsverfahren, Wirbelschicht-Granulation, Sprühgranulation und vergleichbare Technologien geeignet.

Als oberflächenaktive Substanzen kommen vorzugsweise die zuvor genannten anionischen Tenside und Schutzkolloide in Betracht. Die Menge an oberflächenaktiven Substanzen wird in der Regel 1 bis 30 Gew.-%, insbesondere 2 bis 20 Gew.-%, bezogen auf das Gesamtgewicht der erfindungsgemäßen festen Formulierung betragen.

Die Menge an Wirkstoff, d. h. die Gesamtmenge an Topramezon sowie an gegebenenfalls weiteren Wirkstoffen, liegt in derartigen festen Formulierungen typischerweise im Bereich von 10 bis 70 Gew.-%, insbesondere im Bereich von 20 bis 50 Gew.-%, bezogen auf das Gesamtgewicht des nicht-wässrigen Suspensionskonzentrats.

Die folgenden Formulierungsbeispiele verdeutlichen die Herstellung solcher Zubereitungen:
I. Wasserdispergierbares Pulver:
   20 Gewichtsteile der Form I werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Auf diese weise erhält man ein wasserdispergierbares Pulver, welches die Form I enthält.
II. Stäubemittel:
   5 Gewichtsteile der Form I werden mit 95 Gewichtsteilen feinteiligem Kaolin vermischt. Man erhält auf diese Weise ein Stäubemittel, das 5 Gew.-% der Form I enthält.
III. nicht-wässriges Suspensionskonzentrat:
   20 Gewichtsteile der Form I werden mit 2 Gewichtsteilen Calciumsalz der Dodecylbenzolsulfonsäure, 8 Gewichtsteilen Fettalkoholpolyglykolether, 2 Gewichtsteilen Natriumsalz eines Phenolsulfonsäure-Harnstoff-Formaldehyd-Kondensates und 68 Gewichtsteilen eines paraffinischen Mineralöls innig vermischt, z. B. durch gemeinsames Vermahlen oder durch Einwirken starker Scherkräfte. Man erhält ein stabiles nicht-wässriges Suspensionskonzentrat der Form I.
IV. nicht-wässriges Suspensionskonzentrat:
   20 Gew.-Teile der Form I werden unter Zusatz von 10 Gew.-Teilen Dispergier- und Netzmitteln und 70 Gew.-Teilen eines paraffinischen Mineralöls in einer Rührwerkskugelmühle zu einer feinen Wirkstoffsuspension zerkleinert. Man erhält ein stabiles nichtwässriges Suspensionskonzentrat der Form I. Bei der Verdünnung in Wasser ergibt sich eine stabile Suspension des Wirkstoffs. Der Wirkstoffgehalt in der Formulierung beträgt 20 Gew.-%.
V. wässriges Suspensionskonzentrat:
   10 Gewichtsteile der Form I werden in einer Lösung von 17 Gewichtsteilen eines Poly(ethylenglykol)(propylenglykol)blockcopolymeren, 2 Gewichtsteilen eines Phenolsulfonsäure-Formaldehyd-Kondensates und etwa 1 Gewichtsteil sonstigen Hilfsmitteln (Verdicker, Entschäumer) in einem Gemisch aus 7 Gewichtsteilen Propylenglykol und 63 Gewichtsteilen Wasser als wässriges Suspensionskonzentrat formuliert.
VI. wässriges Suspensionskonzentrat:
   20 Gew.-Teile der Form I werden unter Zusatz von 10 Gew.-Teilen Dispergier- und Netzmitteln und 70 Gew.-Teilen Wasser in einer Rührwerkskugelmühle zu einer feinen Wirkstoffsuspension zerkleinert. Bei der Verdünnung in Wasser ergibt sich eine stabile Suspension des Wirkstoffs. Der Wirkstoffgehalt in der Formulierung beträgt 20 Gew.-%.
VII. Wasserdispergierbare und wasserlösliche Granulate
   50 Gew.-Teile der Form I werden unter Zusatz von 50 Gew.-Teilen Dispergier- und Netzmitteln fein gemahlen und mittels technischer Geräte (z. B. Extrusion, Sprühturm, Wirbelschicht) als wasserdispergierbare oder wasserlösliche Granulate formuliert. Bei der Verdünnung in Wasser ergibt sich eine stabile Dispersion oder Lösung des Wirkstoffs. Die Formulierung hat einen Wirkstoffgehalt von 50 Gew.-%.
VIII. Wasserdispergierbare und wasserlösliche Pulver
   75 Gew.-Teile der Form I werden unter Zusatz von 25 Gew.-Teilen Dispergier- und Netzmitteln sowie Kieselsäuregel in einer Rotor-Stator-Mühle vermahlen. Bei der Verdünnung in Wasser ergibt sich eine stabile Dispersion oder Lösung des Wirkstoffs. Der Wirkstoffgehalt der Formulierung beträgt 75 Gew.-%.
IX. Gelformulierungen
   In einer Kugelmühle werden 20 Gew.-Teile der Form I, 10 Gew.-Teile Dispergiermittel, 1 Gew.-Teil Geliermittel und 70 Gew.-Teile Wasser oder eines organischen Lösungsmittels zu einer feinen Suspension vermahlen. Bei der Verdünnung mit Wasser ergibt sich eine stabile Suspension. Der Wirkstoffgehalt der Formulierung beträgt 20 Gew.-%.
X: direkt applizierbare Granulate (GR, FG, GG, MG)
   0,5 Gew.-Teile der Form I werden fein gemahlen und mit 99,5 Gewichtsteilen Trägerstoffe verbunden. Gängige Verfahren sind dabei die Extrusion, die Sprühtrocknung oder die Wirbelschicht. Man erhält dadurch ein Granulat für die Direktapplikation mit 0,5 Gew.-% Wirkstoffgehalt.

Die Applikation der Form I bzw. die der sie enthaltenden herbiziden Mittel erfolgt, sofern die Formulierung nicht bereits gebrauchsfertig ist, in Form wässriger Spritzbrühen. Diese werden durch Verdünnen der vorgenannten, die Form I enthaltenden Formulierungen mit Wasser bereitet. Die Spritzbrühen können auch weitere Bestandteile in gelöster, emulgierter oder suspendierter Form enthalten, beispielsweise Düngemittel, Wirkstoffe anderer herbizider oder wachstumsregulierender Wirkstoffgruppen, weitere Wirkstoffe, z. B. Wirkstoffe zur Bekämpfung von tierischen Schädlingen oder phytopathogenen Pilzen bzw. Bakterien, weiterhin Mineralsalze, welche zur Behebung von Ernährungs- und Spurenelementmängeln eingesetzt werden, sowie nicht-phytotoxische Öle und Ölkonzentrate. In der Regel werden diese Bestandteile vor, während oder nach dem Verdünnen der erfindungsgemäßen Formulierungen der Spritzbrühe zugesetzt.

Die Applikation der Form I bzw. der sie enthaltenden Pflanzenschutzmittel kann im Vorauflauf- oder im Nachauflaufverfahren erfolgen. Sofern Topramezon für gewisse Kulturpflanzen weniger verträglich ist, können Ausbringungstechniken angewandt werden, bei welchen die herbiziden Mittel mit Hilfe der Spritzgeräte so gespritzt werden, dass die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Topramezon betragen je nach Bekämpfungsziel, Jahreszeit, Zielpflanzen und Wachstumsstadium 0,001 bis 3,0, vorzugsweise 0,01 bis 1,0 kg/ha aktive Substanz (a. S.).

In einer weiteren Ausführungsform kann die Applikation der Form I bzw. der sie enthaltenden Pflanzenschutzmittel durch Behandlung von Saatgut erfolgen.

Behandlung von Saatgut umfasst im Wesentlichen alle dem Fachmann geläufigen Techniken (seed dressing, seed coating, seed dusting, seed soaking, seed film coating, seed multilayer coating, seed encrusting, seed dripping, und seed pelleting") basierend auf Topramezon in der Form I bzw. daraus hergestellten Mitteln. Hierbei können die Pflanzenschutzmittel verdünnt oder unverdünnt aufgetragen werden.

Der Begriff Saatgut umfasst Saatgut aller Arten, wie z. B. Körner, Samen, Früchte, Knollen, Stecklinge und ähnliche Formen. Bevorzugt beschreibt der Begriff Saatgut hier Körner und Samen.

Als Saatgut kann Saatgut der oben erwähnten Nutzpflanzen aber auch das Saatgut transgener oder durch herkömmliche Züchtungsmethoden erhaltener Pflanzen eingesetzt werden.

Zur Saatgutbehandlung wird Topramezon üblicherweise in Mengen von 0,001 bis 10 kg pro 100 kg Saatgut eingesetzt.

Zur Verbreiterung des Wirkungsspektrums und zur Erzielung synergistischer Effekte können die Formen I mit zahlreichen Vertretern anderer herbizider oder wachstumsregulierender Wirkstoffgruppen gemischt und gemeinsam ausgebracht werden. Außerdem kann es vorteilhaft sein, Topramezon gemeinsam mit Safenern zu formulieren oder auszubringen.

Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/- Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexenonoximetherderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylhamstoffe, Triazine, Triazinone, Triazolinone, Triazolcarboxamide, Uracile sowie Phenylpyrazoline und Isoxazoline und deren Derivate in Betracht. Insbesondere eignen sich als Mischungspartner Coherbizide wie Terbuthylazin, Bromoxynil, dessen Natriumsalz und dessen Ester mit C₄-C₈-Carbonsäuren, Dicamba, S-Metolachlor oder Pethoxamid, und Safener wie Isoxadifen.

Außerdem kann es von Nutzen sein, die Form I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch Additive wie nicht-phytotoxische Öle und Ölkonzentrate zugesetzt werden.

Die folgenden Abbildungen und Beispiele dienen der Erläuterung der Erfindung und sind nicht einschränkend zu verstehen.

Abbildung 1 zeigt ein Röntgenpulverdifraktogramm der Form I. Das Röntgenpulverdiffraktogramm der Form I wurde mit einem Diffraktometer D-5000 der Fa. Bruker-AXS in Reflexionsgeometrie im Bereich von 2θ = 2° - 40° mit einer Schrittweite von 0,02° unter Verwendung der Cu-K_{α}-Strahlung (1,54178 Å) bei 25 oder 30 °C aufgenommen.

Abbildung 2 zeigt ein Röntgenpulverdifraktogramm der Form II. Das Röntgenpulverdiffraktogramm wurde unter den für Abbildung 1 angegebenen Bedingungen aufgenommen.

Abbildung 3 zeigt ein Röntgenpulverdifraktogramm der Form III. Das Röntgenpulverdiffraktogramm wurde unter den für Abbildung 1 angegebenen Bedingungen aufgenommen.

Abbildung 4 zeigt ein Röntgenpulverdifraktogramm der Form IV. Das Röntgenpulverdiffraktogramm wurde unter den für Abbildung 1 angegebenen Bedingungen aufgenommen.

Abbildung 5 zeigt ein Röntgenpulverdifraktogramm des Solvats VII-S. Das Röntgenpulverdiffraktogramm wurde unter den für Abbildung 1 angegebenen Bedingungen aufgenommen.

Abbildung 6 zeigt die Orientierung der Topramezonmoleküle im Kristallgitter der Form I. Wasserstoffbrückenbindungen sind als gestrichelte Linien gekennzeichnet.

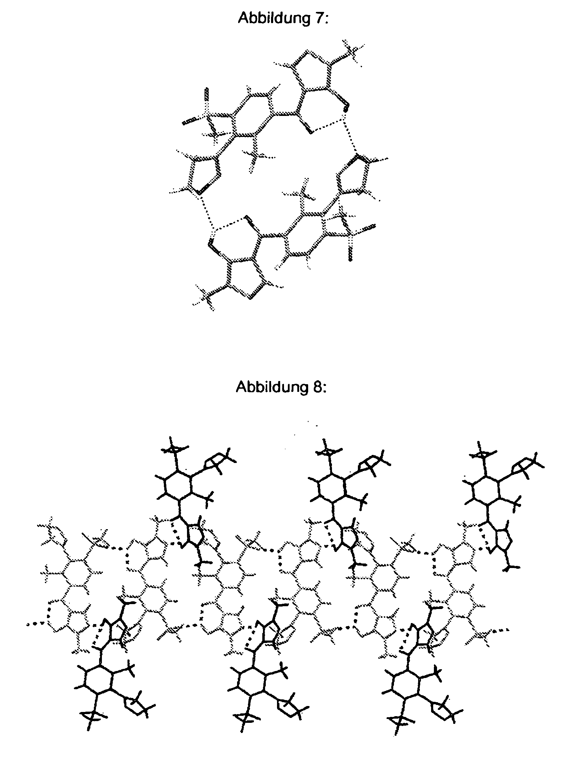

Abbildung 7 zeigt die Orientierung der Topramezonmoleküle im Kristallgitter der Form II. Wasserstoffbrückenbindungen sind als gestrichelte Linien gekennzeichnet.

Abbildung 8 zeigt die Orientierung der Topramezonmoleküle im Kristallgitter der Form IV. Wasserstoffbrückenbindungen sind als gestrichelte Linien gekennzeichnet.

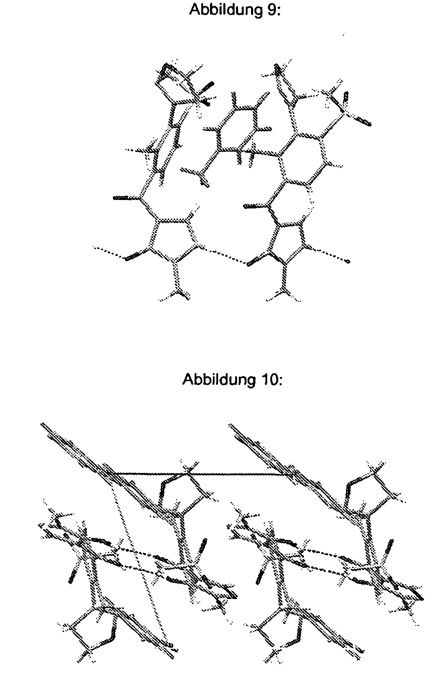

Abbildung 9 zeigt die Orientierung der Topramezonmoleküle und des Toluols im Kristallgitter des Solvats V-S. Wasserstoffbrückenbindungen sind als gestrichelte Linien gekennzeichnet.

Abbildung 10 zeigt die Orientierung der Topramezonmoleküle und des Chlorbenzols im Kristallgitter des Solvats VI-S. Wasserstoffbrückenbindungen sind als gestrichelte Linien gekennzeichnet (Blick entlang der c-Achse der Elementarzelle.

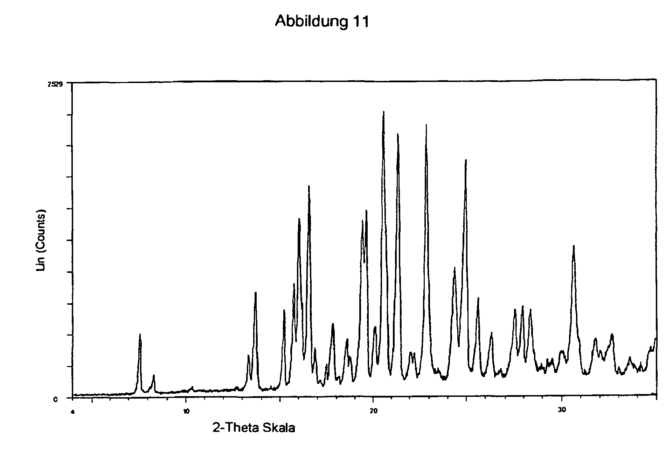

Abbildung 11 zeigt ein Röntgenpulverdifraktogramm der Form VIII. Das Röntgenpulverdiffraktogramm wurde unter den für Abbildung 1 angegebenen Bedingungen aufgenommen.

Die Bestimmung der Schmelzpunkte erfolgte mittels DSC mit einem Mettler Toledo DSC 25 der Fa. Mettler mit einer Aufheizrate von 10 K/min im Bereich von 25° bis +140 °C. Die Probenmenge betrug 5 bis 10 mg.

### Herstellung der Form I des Topramezons

### Beispiel 1:

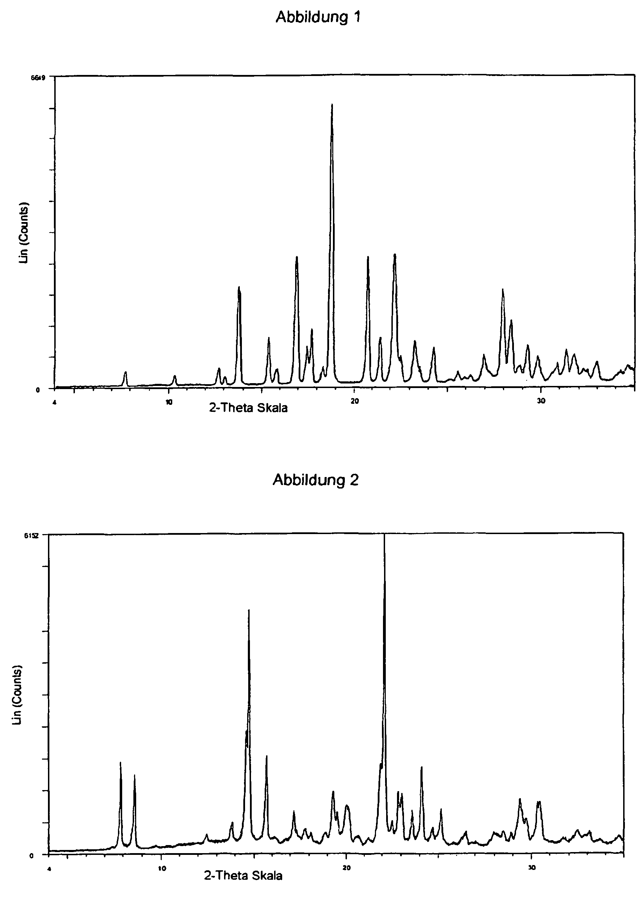

In einem Probengefäß wurden 1 g Topramezon mit einer Reinheit von 96 % in 40 ml Aceton bei etwa 30 °C gelöst. Die Lösung wurde filtriert und das Filtrat wurde anschließend bei einer Temperatur von etwa 30 °C eingeengt, indem man einen Stickstoffstrom über den Flüssigkeitsspiegel leitete. Nach 2 Tagen wurde der Versuch beendet und das erhaltene Kristallisat abgetrennt, getrocknet und analysiert. Die Kristallisationsausbeute lag oberhalb 80 %. Das kristalline Produkt hatte einen Schmelzpunkt von 218 °C. Das Kristallisat wies das in Abbildung 1 gezeigte Röntgenpulverdiffraktogramm auf.

### Beispiel 2:

Der Versuch wurde analog Beispiel 1 durchgeführt, wobei man Ethanol anstelle von Aceton als Lösungsmittel einsetzte. Das kristalline Produkt hatte einen Schmelzpunkt von 218 °C. Ein Röntgenpulverdiffraktogramm belegte das Vorliegen der Form I.

### Beispiel 3:

Der Versuch wurde analog Beispiel 1 durchgeführt, wobei man Isopropanol anstelle von Aceton als Lösungsmittel einsetzte. Das kristalline Produkt hatte einen Schmelzpunkt von 217 °C. Ein Röntgenpulverdiffraktogramm belegte das Vorliegen der Form I.

### Beispiel 4:

In einem Probengefäß wurden 1 g Topramezon mit einer Reinheit von 96 % in 40 ml Aceton unter Rückfluss gelöst. Die heiße Lösung wurde filtriert und erneut zum Rückfluss erhitzt. Das Filtrat wurde anschließend mit 5 bis 10 K/ min auf etwa 25 °C abgekühlt. Anschließend eingeengte man ein, indem man einen Stickstoffstrom über den Flüssigkeitsspiegel leitete. Das erhaltene Kristallisat abgetrennt, getrocknet und analysiert. Das kristalline Produkt hatte einen Schmelzpunkt von 220 °C. Ein Röntgenpulverdiffraktogramm belegte das Vorliegen der Form I.

### Beispiel 5:

In einem Probengefäß wurden 1 g kristallines Topramezon der Form IV mit einer Reinheit von 96 % in 20 ml einer Mischung von 9 Volumenteilen Isopropanol und 1 Volumenteil Wasser unter Rühren 7 Tage suspendiert. Anschließend entfernte man den Überstand durch Zentrifugation. Nach Trocknen über Nacht auf einer Tonplatte erhielt man ein kristallines Material mit einem Schmelzpunkt von 220 °C. Ein Röntgenpulverdiffraktogramm belegte das Vorliegen der Form I.

### Beispiel 6:

Der Versuch wurde analog Beispiel 5 durchgeführt, wobei man Methanol anstelle von Isopropanol als Lösungsmittelbestandteil einsetzte. Das kristalline Produkt hatte einen Schmelzpunkt von 219 °C. Ein Röntgenpulverdiffraktogramm belegte das Vorliegen der Form I.

### Beispiel 7:

In einem Probengefäß wurden 1,4 g kristallines Topramezon der Form eines 1:1-Gemisches der Formen II und III (Reinheit > 96 %) in 20 ml einer Mischung von 1 Volumenteilen Methanol und 9 Volumenteilen Wasser unter Rühren 7 Tage suspendiert. Anschließend entfernte man den Überstand durch Zentrifugation. Nach Trocknen über Nacht erhielt man ein kristallines Material mit einem Schmelzpunkt von 220 °C. Ein Röntgenpulverdiffraktogramm belegte das Vorliegen der Form I.

### Beispiel 8:

Der Versuch wurde analog Beispiel 7 durchgeführt, wobei man Ethylenglykol anstelle von Methanol als Lösungsmittelbestandteil einsetzte. Das kristalline Produkt hatte einen Schmelzpunkt von 220 °C. Ein Röntgenpulverdiffraktogramm belegte das Vorliegen der Form I.

### Beispiel 9:

Der Versuch wurde analog Beispiel 7 durchgeführt, wobei man Topramezon als reine Form II anstelle des Gemisches aus Form II und Form III einsetzte. Das kristalline Produkt hatte einen Schmelzpunkt von 219 °C. Ein Röntgenpulverdiffraktogramm belegte das Vorliegen der Form I.

### Beispiel 10:

In einem Kolben löste man 1,6 g Topramezon mit einer Reinheit von 96 % in 110 ml Methanol unter Rückfluss und filtrierte die heiße Lösung. Anschließend engte man am Rotationsverdampfer bei einem Druck von 65 bis 86 mbar und einer Badtemperatur von etwa 35 °C ein. Das so erhaltene kristalline Produkt hatte einen Schmelzpunkt von 218 °C. Ein Röntgenpulverdiffraktogramm belegte das Vorliegen der Form I.

### Beispiel 11:

Der Versuch wurde analog Beispiel 10 durchgeführt, wobei man Isopropanol anstelle von Methanol als Lösungsmittelbestandteil einsetzte. Das kristalline Produkt hatte einen Schmelzpunkt von 217 °C. Ein Röntgenpulverdiffraktogramm belegte das Vorliegen der Form I.

### Beispiel 12

In einem Druckgefäß löste man 318 g 3-(3-Brom-2-methyl-6-methylsulfonylphenyl)-4,5-dihydroisoxazol in 2565 g Dioxan und gab hierzu unter Rühren 98 g 1-Methyl-5-hydroxypyrazol, 10,5 g Triphenylphosphin, 346 g Kaliumcarbonat und 0,345 g Palladium(II)-chlorid.

Man spülte 3-mal mit CO, erwärmte auf 130 °C und erhöhte dann den CO-Druck auf 15 bar. Die Mischung wurde dann unter kontinuierlicher Nachführung von CO 25 h bei 130 °C und einem Druck von 15 bar gerührt. Anschließend goss man das Reaktionsgemisch auf etwa 4400 g Wasser, filtrierte von ungelöstem ab und destillierte Dioxan/Wasser bis auf ein Endvolumen von etwa 2500 ml ab. Hierzu gab man etwa 1800 g Methanol und fällte das Produkt bei 60 °C durch Zugabe von 430 g konzentrierter Salzsäure. Man kühlte die Suspension auf 20 °C und filtrierte den Feststoff ab und wusch den Feststoff mit Wasser nach. Anschließend suspendierte man den Feststoff in 1920 g Methanol und erhitzte 8 h zum Rückfluss. Anschließend kühlte man die Mischung innerhalb von 4 h auf 20 °C und rührte weitere 2 h bei 20 °C nach. Man filtrierte den Feststoff von der Mutterlauge und wusch 3-mal mit Methanol nach. Der so erhaltene kristalline Feststoff hatte einen Schmelzpunkt von 220 °C. Ein Röntgenpulverdiffraktogramm belegte das Vorliegen der Form I.

### Herstellung der Form II des Topramezons

### Beispiel 13:

In einem Probengefäß wurden 1 g Topramezon mit einer Reinheit von 96 % in 60 ml Toluol unter Rückfluss gelöst. Man kühlte die Lösung auf 20 °C, filtrierte und engte anschließend das Filtrat bei einer Temperatur von etwa 30 °C bis zur Trockne ein, indem man einen Stickstoffstrom über den Flüssigkeitsspiegel leitete. Das erhaltene Kristallisat wurde abgetrennt und analysiert. Das kristalline Produkt hatte einen Schmelzpunkt von 222 bis 223 °C. Das Kristallisat wies das in Abbildung 2 gezeigte Röntgenpulverdiffraktogramm der Form II auf.

### Beispiel 14:

Der Versuch wurde analog Beispiel 13 durchgeführt, wobei man 1,2-Dichlorbenzol anstelle von Toluol als Lösungsmittel einsetzte. Das kristalline Produkt hatte einen Schmelzpunkt von 222 °C. Ein Röntgenpulverdiffraktogramm belegte das Vorliegen der Form II.

### Beispiel 15:

Der Versuch wurde analog Beispiel 13 durchgeführt, wobei man Methylisobutylketon anstelle von Toluol als Lösungsmittel einsetzte. Das kristalline Produkt hatte einen Schmelzpunkt von 222 °C. Ein Röntgenpulverdiffraktogramm belegte das Vorliegen der Form II.

### Herstellung der Form III des Topramezons

### Beispiel 16

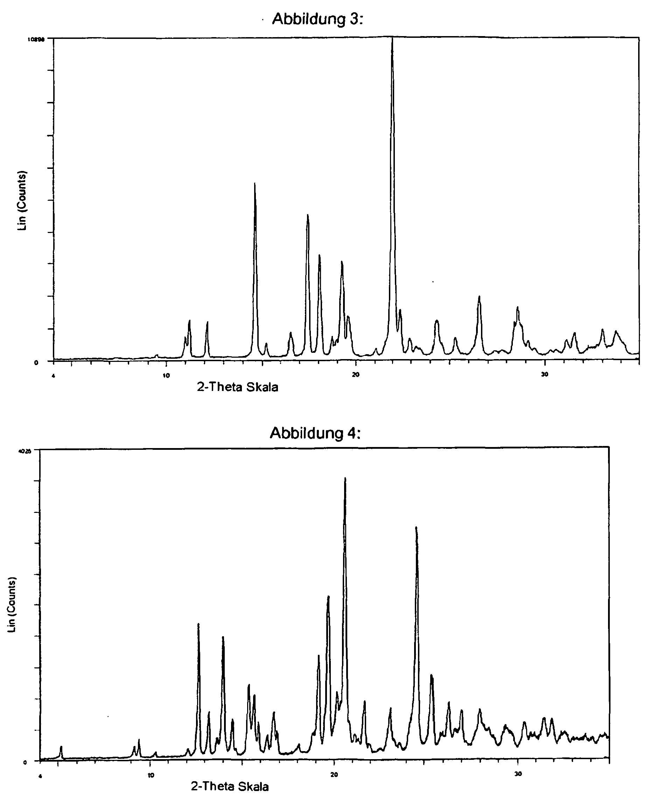

In einem Probengefäß wurden 1 g Topramezon mit einer Reinheit von 96 % in 20 ml Acetophenon unter Rückfluss gelöst. Man filtrierte die heiße Lösung und engte anschließend das Filtrat bei einer Temperatur von etwa 100 °C bis zur Trockne ein, indem man einen Stickstoffstrom über den Flüssigkeitsspiegel leitete. Das erhaltene Kristallisat wurde abgetrennt, getrocknet und analysiert. Das kristalline Produkt hatte einen Schmelzpunkt von 222 °C. Das Kristallisat wies das in Abbildung 3 gezeigte Röntgenpulverdiffraktogramm der Form III auf.

### Beispiel 17:

Der Versuch wurde analog Beispiel 16 durchgeführt, wobei man 1,2-Dichlorbenzol anstelle von Acetophenon als Lösungsmittel einsetzte. Das kristalline Produkt hatte einen Schmelzpunkt von 223 °C. Ein Röntgenpulverdiffraktogramm belegte das Vorliegen der Form III.

### Beispiel 18:

Der Versuch wurde analog Beispiel 16 durchgeführt, wobei man Diethylketon anstelle von Acetophenon als Lösungsmittel einsetzte. Das kristalline Produkt hatte einen Schmelzpunkt von 222 °C. Ein Röntgenpulverdiffraktogramm belegte das Vorliegen der Form III.

### Herstellung einer Formulierung

### Einsatzmaterialien:

- Emulgator 1: EO/PO-Triblockcopolymer mit einem Molekulargewicht von 6500 und einem Propylenoxid-Anteil von 50 Gew.-%.
- Emulgator 2: Natriumsalz eines Phenolsulfonsäure-Formaldehyd-Kondensationsprodukts
- Verdicker: Xanthan-Gum
- Entschäumer: Handelsübliche Polydimethylsiloxan-Füllstoff-Emulsion (Wacker Silikon SRE-PFL) (Aktivgehalt 20 Gew.-%)
- Mikrobiozid: Formulierung, enthaltend Gemisch aus 1,2-Benzisothiazolin-3-on und 2-Methyl-4-isothiazolin-3-on, Aktivgehalt 5 Gew.-% (Aktizide MBS der Thor Chemie GmbH).

### Beispiel 19: wässriges Suspensionskonzentrat von Topramezon der Form I

1. In einem Rührgefäß legte man 400 g entmineralisiertes Wasser vor und gab hierzu nacheinander 60 g 1,2 Propylenglykol, 20 g Emulgator 2 und 166,7 g einer 18 gew-%igen, wässrigen Lösung des Emulgators 1. Man rührte, bis eine homogene klare Lösung erhalten wurde und gab dann hierzu nacheinander 343,9 g kristallines Topramezon der Form I mit einem Topramezon-Gehalt > 98 % und 1 g Entschäumer. Man kühlte die so erhaltene Suspension auf etwa 15 °C und leitete sie dann durch eine Rotor-Stator-Mühle und anschließend unter Kühlung durch eine Kugelmühle, bis die gewünschte Teilchengrößenverteilung erreicht war. Man erhielt auf diese Weise eine wässrige Topramezon-Suspension, worin 80 Gew.-% der Teilchen einen Durchmesser unterhalb 2 µm aufwiesen.
2. In einem Rührgefäß legte man 10 g 1,2 Propylenglykol und 119,4 g entmineralisiertes Wasser vor und gab dann unter Rühren nacheinander 3 g Verdicker und anschließend 2 g des Mikrobiozids. Anschließend gab man die so erhaltene Lösung unter Rühren zu der in Schritt 1 erhaltenen Suspension und gab hiernach weitere 4 g des Entschäumers unter Rühren zu. Auf diese Weise erhielt man eine wässrige Suspension, die etwa 336 g/L Topramezon in der Form I enthielt und die eine Viskosität, bestimmt nach OECD 114 von etwa 60 bis 100 mPa.s aufwies. Die Teilchengrößenverteilung war durch einen d₉₀-Wert von ≤3,5 µm und einem d₅₀-Wert von ≤1,3 µm charakterisiert.

### Beispiel 20: Herstellung eines Suspoemulsionskonzentrats, enthaltend Topramezon der Form I und Dimethenamid-P

Zu 285,7 g entmineralisiertem Wasser gab man unter Rühren 44,4 g 1,2-Propylenglykol, 44,4 g des Emulgators 2 und 66,6 g einer 2 gew.-%igen, wässrigen Lösung des Verdickers, die 1,6 Gew.-% des Biozids enthielt. Zu dieser Lösung gab man unter Rühren bei 23 °C 561 g Dimethenamid P und rührte so lange, bis man eine stabile Emulsion erhielt. Zu der so erhaltenen Emulsion gab man anschließend 107,6 g des in Beispiel 19 hergestellten Suspensionskonzentrats und rührte weitere 10 Minuten.

Auf diese Weise erhielt man eine wässrige Suspoemulsion, die einen Gehalt an Dimethenamid-P von etwa 538 g und einen Gehalt an Topramezon in der Form I von etwa 32 g/L aufinries. Die Dichte lag bei etwa 1.11 g/cm³. Die Viskosität, bestimmt mit einem Rotationsviskosimeter nach der OECD-Testvorschrift 114 lag bei etwa 70 bis 90 mPa.s. Der d₉₀-Wert lag unterhalb 7 µm und der d₅₀-Wert lag unterhalb 1,5 µm. Der pH-Wert einer etwa 1 gew.-%igen Verdünnung in entmineralisiertem Wasser lag im Bereich von etwa 2,5 bis 4,5.

## Patentansprüche

1. Kristalline Form I des [3-(4,5-Dihydro-3-isoxazolyl)-2-methyl-4-(methylsulfonyl)-phenyl]-(5-hydroxy-1-methyl-1 H-pyrazol-4-yl)methanons, die in einem Röntgenpulverdiffraktogramm bei 30 °C und Cu-Kα-Strahlung wenigstens 5 der folgenden, als 2θ-Werte angegebenen Reflexe zeigt: 7,7 ± 0,2°, 10,3 ± 0,2°, 12,7 ± 0,2°, 13,8 ± 0,2°, 16,9 ± 0,2°, 18,8 ± 0,2°, 20,7 ± 0,2°, 22,2 ± 0,2°, 28,0 ± 0,2°, 31,4 ± 0,2°.

2. [3-(4,5-Dihydro-3-isoxazolyl)-2-methyl-4-(methylsulfonyl)phenyl]-(5-hydroxy-1-methyl-1 H-pyrazol-4-yl)methanon, bestehend zu wenigstens 90 Gew.-% aus der kristallinen Form I.

3. Verfahren zur Herstellung der kristallinen Form I gemäß einem der Ansprüche 1 oder 2, umfassend:
i) Bereitstellung einer Lösung von [3-(4,5-Dihydro-3-isoxazolyl)-2-methyl-4-(methylsulfonyl)phenyl]-(5-hydroxy-1-methyl-1 H-pyrazol-4-yl)methanon in einem polaren organischen Lösungsmittel, das unter C₁-C₄-Alkanolen, C₂-C₄-Alkandiolen, Aceton und deren Mischung mit Wasser ausgewählt ist,
ii) Bewirken einer Kristallisation von [3-(4,5-Dihydro-3-isoxazolyl)-2-methyl-4-(methylsulfonyl)phenyl]-(5-hydroxy-1-methyl-1H-pyrazol-4-yl)methanon.

4. Verfahren zur Herstellung der kristallinen Form I gemäß einem der Ansprüche 1 oder 2, umfassend:
i) Bereitstellung einer Suspension von [3-(4,5-Dihydro-3-isoxazolyl)-2-methyl-4-(methylsulfonyl)phenyl]-(5-hydroxy-1-methyl-1H-pyrazol-4-yl)methanon in einem polaren organischen Lösungsmittel, das unter C₁-C₄-Alkanoten, C₂-C₄-Alkandiolen, Aceton und deren Mischung mit Wasser ausgewählt ist,
ii) Bewegen des suspendierten Materials in der Suspension.

5. Pflanzenschutzmittel, enthaltend [3-(4,5-Dihydro-3-isoxazolyl)-2-methyl-4-(methylsulfonyl)phenyl]-(5-hydroxy-1-methyl-1H-pyrazol-4-yl)methanon, das zu wenigstens 90 Gew.-% aus der kristallinen Form I gemäß Anspruch 1 besteht, und ein oder mehrere, für die Formulierung von Pflanzenschutzmitteln übliche Hilfsmittel.

6. Pflanzenschutzmittel gemäß Anspruch 5 in Form eines wässrigen Suspensionskonzentrats.

7. Pflanzenschutzmittel gemäß Anspruch 5 in Form eines nicht-wässrigen Suspensionskonzentrats.

8. Pflanzenschutzmittel gemäß Anspruch 5 in Form eines in Wasser dispergierbaren Pulvers oder Granulats.

9. Verfahren zur Bekämpfung von unerwünschtem Pflanzenwuchs, **dadurch gekennzeichnet, dass** man [3-(4,5-Dihydro-3-isoxazolyl)-2-methyl-4-(methylsulfonyl)phenyl]-(5-hydroxy-1-methyl-1H-pyrazol-4-yl)methanon, bestehend zu wenigstens 90 Gew.-% aus der kristallinen Form I gemäß Anspruch 1, auf Pflanzen, deren Lebensraum und/oder auf Samen einwirken lässt.

## Claims

1. A crystalline form I of [3-(4,5-dihydro-3-isoxazolyl)-2-methyl-4-(methylsulfonyl)phenyl]-(5-hydroxy-1-methyl-1*H*-pyrazol-4-yl)methanone, which in an X-ray powder diffractogram at 30°C with Cu-Kα radiation displays at least 5 of the following reflections, stated as 2θ values: 7.7 ± 0.2°, 10.3 ± 0.2°, 12.7 ± 0.2°, 13.8 ± 0.2°, 16.9 ± 0.2°, 18.8 ± 0.2°, 20.7 ± 0.2°, 22.2 ± 0.2°, 28.0 ± 0.2° and 31.4 ± 0.2°.

2. [3-(4,5-dihydro-3-isoxazolyl)-2-methyl-4-(methylsulfonyl)phenyl]-(5-hydroxy-1-methyl-1*H-*pyrazol-4-yl)methanone, being composed of at least 90 % by weight of the crystalline form I.

3. A process for the preparation of the crystalline form I according to one of claims 1 or 2, comprising:
i) Preparation of a solution of [3-(4,5-dihydro-3-isoxazolyl)-2-methyl-4-(methylsulfonyl)phenyl]-(5-hydroxy-1-methyl-1*H*-pyrazol-4-yl)methanone in a polar organic solvent, which is selected from C₁-C₄ alkanols, C₂-C₄ alkanediols, acetone and the mixture thereof with water,
ii) Performing a crystallization of [3-(4,5-dihydro-3-isoxazolyl)-2-methyl-4- (methylsulfonyl)phenyl]-(5-hydroxy-1-methyl-1*H-*pyrazol-4-yl)methanone.

4. A process for the preparation of the crystalline form I according to one of claims 1 or 2, comprising:
i) Preparation of a suspension of [3-(4,5-dihydro-3-isoxazolyl)-2-methyl-4-(methylsulfonyl)-phenyl]-(5-hydroxy-1-methyl-1*H*-pyrazol-4-yl)methanone in a polar organic solvent, which is selected from C₁-C₄ alkanols, C₂-C₄ alkanediols, acetone and the mixture thereof with water,
ii) Stirring of the suspended material in the suspension.

5. A plant protection agent, comprising [3-(4,5-dihydro-3-isoxazolyl)-2-methyl-4-(methylsulfonyl)phenyl]-(5-hydroxy-1-methyl-1*H-*pyrazol-4-yl)methanone, which is composed of at least 90 % by weight of the crystalline form I according to claim 1, and one or more additives conventional for the formulation of plant protection agents.

6. The plant protection agent according to claim 5 in the form of an aqueous suspension concentrate.

7. The plant protection agent according to claim 5 in the form of a non-aqueous suspension concentrate.

8. The plant protection agent according to claim 5 in the form of a powder or granules dispersible in water.

9. A method for the control of undesired plant growth, wherein plants, the habitat and/or the seeds thereof are subjected to the action of [3-(4,5-dihydro-3-isoxazolyl)-2-methyl-4-(methylsulfonyl)-phenyl]-(5-hydroxy-1-methyl-1*H*-pyrazol-4-yl)methanone, being composed of at least 90 % by weight of the crystalline form I according to claim 1.

## Revendications

1. Forme cristalline de la [3-(4,5-dihydro-3-isoxazolyl)-2-méthyl-4-(méthylsulfonyl)phényl]-(5-hydroxy-1-méthyl-1H-pyrazol-4-yl)méthanone, qui présente dans un diagramme de diffraction des rayons X sur poudre à 30 °C et dans la raie Kα du cuivre au moins 5 des réflexions suivantes, indiquées en tant que valeurs 2θ : 7,7 ± 0,2°, 10,3 ± 0,2°, 12,7 ± 0,2°, 13,8 ± 0,2°, 16,9 ± 0,2°, 18,8 ± 0,2°, 20,7 ± 0,2°, 22,2 ± 0,2°, 28,0 ± 0,2°, 31,4 ± 0,2°.

2. [3-(4,5-dihydro-3-isoxazolyl)-2-méthyl-4-(méthylsulfonyl)phényl]-(5-hydroxy-1-méthyl-1H-pyrazol-4-yl)méthanone, consistant à raison d'au moins 90 % en poids en la forme cristalline I.

3. Procédé pour la préparation de la forme cristalline I selon l'une quelconque des revendications 1 et 2, comprenant :
i) la production d'une solution de [3-(4,5-dihydro-3-isoxazolyl)-2-méthyl-4-(méthylsulfonyl)phényl]-(5-hydroxy-1-méthyl-1H-pyrazol-4-yl)méthanone dans un solvant organique polaire, qui est choisi parmi des alcanols en C₁-C₄, des alcane (C₂-C₄) diols, l'acétone et leur mélange avec l'eau,
ii) la production d'une cristallisation de [3-(4,5-dihydro-3-isoxazolyl)-2-méthyl-4-(méthylsulfonyl)- phényl]-(5-hydroxy-1-méthyl-1H-pyrazol-4-yl)-méthanone.

4. Procédé pour la préparation de la forme cristalline I selon l'une quelconque des revendications 1 et 2, comprenant :
i) la production d'une suspension de [3-(4,5-dihydro-3-isoxazolyl)-2-méthyl-4-(méthylsulfonyl)phényl]-(5-hydroxy-1-méthyl-1H-pyrazol-4-yl)méthanone dans un solvant organique polaire, qui est choisi parmi des alcanols en C₁-C₄, des alcane(C₂-C₄)diols, l'acétone et leur mélange avec l'eau,
ii) la mise en mouvement de la substance en suspension dans la suspension.

5. Produit phytosanitaire, contenant de la [3-(4,5-dihydro-3-isoxazolyl)-2-méthyl-4-(méthylsulfonyl)-phényl]-(5-hydroxy-1-méthyl-1H-pyrazol-4-yl)méthanone, qui consiste à raison d'au moins 90 % en poids en la forme cristalline I selon la revendication 1, et un ou plusieurs adjuvants usuels pour la formulation de produits phytosanitaires.

6. Produit phytosanitaire selon la revendication 5, sous forme d'un concentré aqueux de suspension.

7. Produit phytosanitaire selon la revendication 5, sous forme d'un concentré non aqueux de suspension.

8. Produit phytosanitaire selon la revendication 5, sous forme d'un produit granulé ou d'une poudre dispersable dans l'eau.

9. Procédé pour la lutte contre la croissance indésirable de plantes, **caractérisé en ce qu'**on fait agir sur des plantes, leur habitat et/ou sur des graines de la [3-(4,5-dihydro-3-isoxazolyl)-2-méthyl-4-(méthylsulfonyl)phényl]-(5-hydroxy-1-méthyl-1H-pyrazol-4-yl)méthanone, consistant à raison d'au moins 90 % en poids en la forme cristalline I selon la revendication 1.
